# EUROPÄISCHE PATENTSCHRIFT

(11) **EP 1 601 335 B1**
(45) Veröffentlichungstag und Bekanntmachung des Hinweises auf die Patenterteilung: **19.03.2008**
(21) Anmeldenummer: 04706670.9
(22) Anmeldetag: 30.01.2004
(51) Int. Cl.: A61K 8/49, A61Q 5/10

(54) **MITTEL UND VERFAHREN ZUM GLEICHZEITIGEN AUFHELLEN UND FÄRBEN VON KERATINFASERN**
MEANS AND METHOD FOR THE SIMULTANEOUS BLEACHING AND DYEING OF KERATIN FIBRES
AGENT ET PROCEDE D'ECLAIRCISSEMENT ET DE COLORATION SIMULTANES DES FIBRES KERATINIQUES

(30) Priorität: 05.03.2003 DE 10309522
(43) Veröffentlichungstag der Anmeldung: 07.12.2005
(73) Patentinhaber: Wella Aktiengesellschaft, 64274 Darmstadt (DE)
(72) Erfinder: PASQUIER, Cécile, CH-1723 Marly (CH); KIENER, Caroline, CH-1731 Ependes (CH); BRAUN, Hans-Jürgen, CH-3182 Ueberstorf (CH)
(74) Vertreter: Bockhorst, Matthias
(86) Internationale Anmeldenummer: PCT/EP2004/000816
(87) Internationale Veröffentlichungsnummer: WO 2004/078152

(56) Entgegenhaltungen:
- WO-A-00/76469
- WO-A-02/074268
- WO-A-03/042199
- DE-B- 1 049 381
- FR-A- 1 599 968
- GB-A- 1 554 331

## Beschreibung

Gegenstand der vorliegenden Erfindung ist ein Mittel zum gleichzeitigen Aufhellen und Färben von Keratinfasern, wie zum Beispiel Seide, Wolle oder Haaren und insbesondere menschlichen Haaren, auf der Basis einer Entwicklersubstanz-Kupplersubstanz-Kombination, welches mindestens ein heterozyklisches Hydrazon-Derivat als Enfwicklersubstanz, und mindestens ein Persulfatsalz und Wasserstoffperoxid oder dessen Additionsverbindungen enthält, und einen basischen pH aufweist sowie ein Verfahren zum gleichzeitigen Aufhellen und Färben von Keratinfasern unter Verwendung des vorgenannten Mittels.

Haarfärbemittel werden je nach zu färbender Ausgangshaarfarbe und gewünschtem Endresultat hauptsächlich in die Gruppe der Oxidationsfärbemittel oder der Tönungen unterteilt. Oxidationsfärbemittel eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe, wie zum Beispiel Nitrofarbstoffe, können aufgrund ihrer geringen Größe in das Haar eindringen und es -zumindest in den äusseren Bereichen- direkt anfärben. Derartige Tönungen sind sehr haarschonend und überstehen in der Regel 6 bis 8 Haarwäschen.

Direktziehende Farbstoffe werden ebenfalls oft in oxidativen Färbemitteln zur Erzeugung bestimmter Nuancen beziehungsweise zur Intensivierung der Farbe eingesetzt. Mit den üblichen Oxidationsfärbemittel ist in der Regel eine Aufhellung von ein bis zwei Tonstufen möglich. Ebenfalls ist es möglich, zum gleichzeitigen Aufhellen und Färben von Keratinfasern eine Kombination aus oxidationsstabilen direktziehenden Farbstoffen und Oxidationsmitteln zu verwenden. Derartige Mittel werden zum Beispiel in der WO 97/20545 oder in der WO 02/074270 beschrieben. Im Vergleich zu mit Oxidationsfärbemitteln erzielten Färbungen besitzen mit Direktziehern erhaltene Färbungen in der Regel jedoch eine geringere Haltbarkeit.

Aufgabe der vorliegenden Erfindung ist es daher, ein Färbemittel für Keratinfasern zur Verfügung zu stellen, das bei basischen pH-Werten beständige Färbungen ergibt, eine Aufhellung bis zu vier Tonstufen ermöglicht und sowohl die Nuancierung von Modetönen als auch Naturtönen erlaubt.

Gegenstand der vorliegenden Erfindung ist daher ein gebrauchsfertiges Mittel zum gleichzeitigen Aufhellen und Färben von Keratinfasern (A) auf der Basis einer Entwicklersubstanz-Kupplersubstanz-Kombination, wie zum Beispiel Wolle, Seide oder Haaren und insbesondere menschlichen Haaren, welches einen basischen pH-Wert aufweist und dadurch gekennzeichnet ist, dass es (a) mindestens ein heterozyklisches Hydrazon-Derivat der Formel (I) oder dessen physiologisch verträgliches Salz, 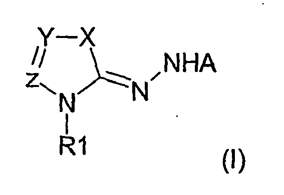 worin
**X** gleich Sauerstoff, Schwefel oder N-R2 ist,
**Y** gleich C-R3 oder Stickstoff ist und
**Z** gleich C-R4 oder Stickstoff ist,
mit der Bedingung, dass der heterozyklische Teil der Verbindung der Formel (I) maximal drei Heteroatome enthält;
**A** Wasserstoff, eine Acetylgruppe, eine Trifluoracetylgruppe, eine Formylgruppe, eine (C₁-C₆)-Alkylsulfonylgruppe oder eine Arylsulfonylgruppe darstellt;
**R1** und **R2** gleich oder verschieden sein können, und unabhängig voneinander eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine Amino-(C₁-C₁₂)-alkylgruppe, eine Sulfonsäure-(C₁-C₁₂)-alkylgruppe, eine Formylgruppe, eine C(O)-(C₁-C₁₂)-Alkyl-gruppe, eine C(O)-Phenylgruppe, eine C(O)NH-Alkylgruppe, eine C(O)NH-Phenylgruppe, eine substituierte oder unsubstituierte Phenylgruppe oder eine Benzylgruppe darstellen;
**R3** und **R4** gleich oder verschieden sein können und unabhängig voneinander Wasserstoff, ein Halogenatom (F, Cl, Br, J), eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₁₂)-Alkoxygruppe, eine Cyanogruppe, eine Nitrogruppe, eine Aminogruppe, eine (C₁-C₁₂)-Alkyl-aminogruppe, eine (C₁-C₁₂)-Dialkylaminogruppe, eine Carbonsäure, eine C(O)O-(C₁-C₁₂)-Alkylgruppe, eine substituierte oder unsubstituierte C(O)O-Phenylgruppe, eine substituierte oder unsubstituierte Phenylgruppe oder eine Naphtylgruppe darstellen;
und wenn **Y** und **Z** gleich C-R3 und C-R4 sind, **R3** und **R4** gemeinsam mit dem Restmolekül ein heterozyklisches oder carbozyklisches, gesättigtes oder ungesättigtes, substituiertes oder unsubstituiertes Ringsystem bilden;
(b) mindestens eine an sich bekannte Kupplersubstanz oder deren physiologisch verträgliches Salz, und
(c) als Oxidationsmittel eine Kombination aus mindestens einem Persulfatsalz und Wasserstoffperoxid und/oder dessen Additionsverbindungen enthält.

Bevorzugt sind Hydrazonderivate der Formel (I) oder deren physiologisch verträgliche Salze, bei denen gilt:
(i) **X** ist gleich Schwefel, **Y** ist gleich C-R3, **Z** ist gleich C-R4 und **A** stellt ein Wasserstoffatom dar, oder
(ii) **X** ist gleich N-R2, **Y** ist gleich Stickstoff und **A** stellt ein Wasserstoffatom;
wobei Hydrazonderivate der Formel (I) oder deren physiologisch verträgliche Salze mit **X** gleich Schwefel, **Y** gleich C-R3, Z gleich C-R4 und **A** gleich Wasserstoff besonders bevorzugt sind.

Als Beispiel für die Verbindungen der Formel (1) können die folgenden Verbindungen gennant werden:
3-Methyl-2(3H)-thiazolon-hydrazon,
3,4-Dimethyl-2(3H)-thiazolon-hydrazon,
4-tert-Butyl-3-methyl-2(3H)-thiazolon-hydrazon,
3-Methyl-4-phenyl-2(3H)-thiazolon-hydrazon,
3-Methyl-4-(4-tolyl)-2(3H)-thiazolon-hydrazon,
4-(4-Methoxy)phenyl-3-methyl-2(3H)-thiazofon-hydrazon,
4-(4-Ethoxy)phenyl-3-methyl-2(3H)-thiazolon-hydrazon,
4-(4-Bromphenyl)-3-methyl-2(3H)-thiazolon-hydrazon,
4-(3-Bromphenyl)-3-methyl-2(3H)-thiazolon-hydrazon,
4-(4-Chlorphenyl)-3-methyl-2(3H)-thiazolon-hydrazon,
4-(3-Chlorphenyl)-3-methyl-2(3H)-thiazolon-hydrazon,
3-Methyl-4-(4-nitrophenyl)-2(3H)-thiazolon-hydrazon,
3-Methyl-4-(3-nitrophenyl)-2(3H)-thiazolon-hydrazon,
4-([1,1'-Biphenyl]-4-yl)-3-methyl-2(3H)-thiazolon-hydrazon,
3-Methyl-4-(2-naphthalenyl)-2(3H)-thiazolon-hydrazon,
2-Hydrazono-2,3-dihydro-3-methyl-4-thiazolcarbonsäureethylester,
3,4,5-Trimethyl-2(3H)-thiazolon-hydrazon,
3,4-Dimethyl-5-phenyl-2(3H)-thiazolon-hydrazon,
3,5-Dimethyl-4-phenyl-2(3H)-thiazolon-hydrazon,
3-Methyl-4,5-diphenyl-2(3H)-thiazolon-hydrazon,
5-Ethyl-3-methyl-4-phenyl-2(3H)-thiazolon-hydrazon,
4-(4-Bromphenyl)-3-methyl-5-phenyl-2(3H)-thiazolon-hydrazon,
3-Methyl-5-phenyl-4-(4-tolyl)-2(3H)-thiazolon-hydrazon,
5-(4-Chlorphenyf)-4-phenyl-3-methyl-2(3H)-thiazolon-hydrazon,
5-(4-Chlorphenyl)-4-(4-methoxyphenyl)-3-methyl-2(3H)-thiazolon-hydrazon,
2-Hydrazono-2,3-dihydro-3,4-dimethyl-4-thiazolcarbonsäureethylester,
4-Amino-2-hydrazono-2,3-dihydro-3-methyl-5-thiazolcarbonitril,
3-Ethyl-4,5-dimethyl-2(3H)-thiazolon-hydrazon,
2-Hydrazono-2,3-dihydro-3-ethyl-4-methyl-thiazolcarbonsäureethylester,
5-Methyl-3-(1-methylethyl)-4-phenyl-2(3H)-thiazolon-hydrazon,
4,5-Dimethyl-3-(1-methylethyl)- 2(3H)-thiazolon-hydrazon,
3-(1-Methylethyl)-4,5-diphenyl-2(3H)-thiazolon-hydrazon,
4,5-Dimethyl-3-propyl-2(3H)-thiazolon-hydrazon,
4,5-Diphenyl-3-propyl-2(3H)-thiazolon-hydrazon,
3-Butyl-4,5-dimethyl-2(3H)-thiazolon-hydrazon,
3-Butyl-4,5-diphenyl-2(3H)-thiazolon-hydrazon,
4,5-Dimethyl-3-(2-methylpropyl)- 2(3H)-thiazolon-hydrazon,
3-(2-Methylpropyl)-4,5-diphenyl-2(3H)-thiazolon-hydrazon,
3-Hydroxyethyl-2(3H)-thiazolon-hydrazon,
3-Hydroxyethyl-4-methyl-2(3H)-thiazolon-hydrazon,
3-Hydroxyethyl-4,5-dimethyl-2(3H)-thiazolon-hydrazon,
3-Aminoethyl-2(3H)-thiazolon-hydrazon,
3-Aminoethyl-4-methyl-2(3H)-thiazolon-hydrazon,
3-Aminoethyl-4,5-dimethyl-2(3H)-thiazolon-hydrazon,
3,4-Diphenyl-2(3H)-thiazolon-hydrazon,
4-Methyl-3-phenyl-2(3H)-thiazolon-hydrazon,
4-p-Biphenylyl-3-phenyl-2(3H)-thiazolon-hydrazon,
4-(4-Methoxy)phenyl-3-phenyl-2(3H)-thiazolon-hydrazon,
4-tert-Butyl-3-phenyl-2(3H)-thiazolon-hydrazon,
4,5-Dimethyl-3-phenyl-2(3H)-thiazolon-hydrazon,
5-Methyl-3,4-diphenyl-2(3H)-thiazolon-hydrazon,
3,4,5-Triphenyl-2(3H)-thiazolon-hydrazon,
4,5-Dimethyl-3-(phenylmethyl)-2(3H)-thiazolon-hydrazon,
3-(2-Propenyl)-2(3H)-thiazolon-hydrazon,
4-Methyl-3-(2-propenyl)-2(3H)-thiazolon-hydrazon,
4-tert-Butyl-3-(2-propenyl)-2(3H)-thiazolon-hydrazon,
4-Phenyl-3-(2-propenyl)-2(3H)-thiazolon-hydrazon,
4,5-Dimethyl-3-(2-propenyl)-2(3H)-thiazolon-hydrazon,
4,5-Diphenyl-3-(2-propenyl)-2(3H)-thiazolon-hydrazon,
4,5-Dimethyl-3-(phenylmethyl)-2(3H)-thiazolon-hydrazon,
2-Hydrazono-2,3-dihydro-3-[(phenylamino)carbonyl]-4-methylthiazolcarbonsäureethylester,
3-Methyl-4,5,6,7-tetrahydro-2(3H)-benzothiazolon-hydrazon,
3-Methyl-2(3H)-benzothiazolon-hydrazon,
3,6-Dimethyl-2(3H)-benzothiazolon-hydrazon,
6-Chlor-3-methyl-2(3H)-benzothiazolon-hydrazon,
7-Chlor-3-methyl-2(3H)-benzothiazolon-hydrazon,
6-Hydroxy-3-methyl-2(3H)-benzothiazolon-hydrazon,
5-Methoxy-3-methyl-2(3H)-benzothiazolon-hydrazon,
7-Methoxy-3-methyl-2(3H)-benzothiazolon-hydrazon,
5,6-Dimethoxy-3-methyl-2(3H)-benzothiazolon-hydrazon,
5-Ethoxy-3-methyl-2(3H)-benzothiazolon-hydrazon,
6-Ethoxy-3-methyl-2(3H)-benzothiazolon-hydrazon,
3-Methyl-5-nitro-2(3H)-benzothiazolon-hydrazon,
3-Methyl-6-nitro-2(3H)-benzothiazolon-hydrazon,
5-Acetamido-3-methyl-2(3H)-benzothiazolon-hydrazon,
6-Acetamido-3-methyl-2(3H)-benzothiazolon-hydrazon,
5-Anilino-3-methyl-2(3H)-benzothiazolon-hydrazon,
6-Anilino-3-methyl-2(3H)-benzothiazolon-hydrazon,
2-Hydrazono-2,3-dihydro-3-methyl-6-benzothiazol-carbonsäure,
2-Hydrazono-2,3-dihydro-3-methyl-4-benzothiazol-sulfonsäure,
2-Hydrazono-2,3-dihydro-3-methyl-5-benzothiazol-sulfonsäure,
2-Hydrazono-2,3-dihydro-3-methyl-6-benzothiazol-sulfonsäure,
2-Hydrazono-2,3-dihydro-3-methyl-7-benzothiazol-sulfonsäure,
2-Hydrazono-2,3-dihydro-N,N,3-trimethyl-6-benzothiazol-sulfonsäureamid,
[(2-Hydrazono-2,3-dihydro-3-methyl-6-benzothiazolyl)oxy]essigsäure-hydrazid,
3-Methyl-naphtho[2,3-d]thiazol-2(3H)-on-hydrazon,
3-Ethyl-2(3H)-benzothiazolon-hydrazon,
6-Ethoxy-3-ethyl-2(3H)-benzothiazolon-hydrazon,
3-Propyl-2(3H)-benzothiazolon-hydrazon,
3-Butyl-2(3H)-benzothiazolon-hydrazon,
3-Hexyl-2(3H)-benzothiazolon-hydrazon,
3-Hydroxyethyl-2(3H)-benzothiazolon-hydrazon,
3-Aminoethyl-2(3H)-benzothiazolon-hydrazon,
3-p-Methylbenzyl-2(3H)-benzothiazolon-hydrazon,
2-Hydrazono-2,3-dihydro-3-(2-hydroxyethyl)-6-benzothiazol-carbonsäure,
2-Hydrazono-2,3-dihydro-6-methoxy-3(2H)-benzothiazol-propan-sulfonsäure,
6-Hexadecyloxy-2-hydrazono-3(2H)-benzothiazol-propan-sulfonsäure,
2-Oxo-3-benzothiazolin-essigsäureethylester-hydrazon,
3-Acetyl-2(3H)-benzothiazolon-hydrazon,
2-Hydrazono-3(2H)-benzothiazol-carboxaldehyd,
3-Methyl-2(3H)-oxazolon-hydrazon,
3-Phenyl-2(3H)-oxazolon-hydrazon,
3-Methyl-2(3H)-benzoxazolon-hydrazon
3-Phenyl-2(3H)-benzoxazolon-hydrazon,
1,3-Dimethyl-4-imidazolin-2-on-hydrazon,
1,3-Diethyl-4-imidazolin-2-on-hydrazon,
1,3-Dihydroxyethyl-4-imidazolin-2-on-hydrazon,
1,3-Diaminoethyl-4-imidazolin-2-on-hydrazon,
1,3-Dimethyl-4-methoxy-4-imidazolin-2-on-hydrazon,
1,3,4-Trimethyl-4-imidazolin-2-on-hydrazon,
1,3-Dimethyl-4-phenyl-4-imidazolin-2-on-hydrazon,
4-Carboxy-1,3-dimethyl-4-imidazolin-2-on-hydrazon,
4-Amino-1,3-dimethyl-4-imidazolin-2-on-hydrazon,
1,3-Dimethyl-4.-dimethylamino-4-imidazolin-2-on-hydrazon,
1,3-Dimethyl-2-benzimidazolinon-hydrazon,
1,3-Diethyl-2-benzimidazolinon-hydrazon,
1,3-Dihydroxyethyl-2-benzimidazolinon-hydrazon,
1,3-Diaminoethyl-2-benzimidazolinon-hydrazon,
1,3,5-Trimethyl-2-benzimidazolinon-hydrazon,
5-Methoxy-1,3-dimethyl-2-benzimidazolinon-hydrazon,
5-Brom-1,3-dimethyl-2-benzimidazolinon-hydrazon,
4,6-Dibrom-1,3-dimethyl-2-benzimidazolinon-hydrazon,
5-Chlor-1,3-dimethyl-2-benzimidazolinon-hydrazon,
1,3-Dimethyl-5-nitro-2-benzimidazolinon-hydrazon,
1,3-Dimethyl-6-nitro-2-benzimidazolinon-hydrazon,
1,4-Dimethyl-Δ2-1,2,4-triazolin-5-on-hydrazon,
1,4-Dihydroxyethyl-Δ2-1,2,4-triazolin-5-on-hydrazon,
1,4-Diaminoethyl-Δ2-1,2,4-triazolin-5-on-hydrazon,
1,3,4-Trimethyl-Δ2-1,2,4-triazolin-5-on-hydrazon,
1,4-Dimethyl-3-phenyl-Δ2-1,2,4-triazolin-5-on-hydrazon,
1,4-Dimethyl-3-methoxy-Δ2-1,2,4-triazolin-5-on-hydrazon,
1,4-Dimethyl-3-dimethylamino-Δ2-1,2,4-triazolin-5-on-hydrazon,
4-Carboxy-1,4-dimethyl-Δ2-1,2,4-triazolin-5-on-hydrazon,
4-Amino-1,4-dimethyl-Δ2-1,2 ,4-triazolin-5-on-hydrazon,
4-Butyl-1-methyl-3-phenyl-Δ2-1,3,4-triazolin-5-on-hydrazon,
4-Methyl-Δ2-1,3,4-thiadiazolin-5-on-hydrazon,
4-Hydroxyethyl-Δ2-1,3,4-thiadiazolin-5-on-hydrazon,
4-Aminoethyl-Δ2-1,3,4-thiadiazolin-5-on-hydrazon,
4-Methyl-2-phenyl-Δ2-1,3,4-thiadiazolin-5-on-hydrazon,
2-Methoxy-4-methyl-Δ2-1,3,4-thiadiazolin-5-on-hydrazon,
2-Anilino-4-methyl-Δ2-1,3,4-thiadiazolin-5-on-hydrazon,
2-Amino-4-methyl-Δ2-1,3,4-thiadiazolin-5-on-hydrazon,
2-Dimethylamino-4-methyl-Δ2-1,3,4-thiadiazolin-5-on-hydrazon,
4-Methyl-2-(methylthio)-Δ2-1,3,4-thiadiazolin-5-on-hydrazon,
4-(5-Hydrazono-4,5-dihydro-4-methyl-1,3,4-thiadiazol-2-yl)-benzensulfonyl fluorid,
4-Methyl-Δ2-1,2,4-thiadiazolin-5-on-hydrazon,
4-Hydroxyethyl-Δ2-1,2,4-thiadiazolin-5-on-hydrazon,
4-Aminoethyl-Δ2-1,2,4-thiadiazolin-5-on-hydrazon,
4-Methyl-3-phenyl-Δ2-1,2,4-thiadiazolin-5-on-hydrazon,
3-Methoxy-4-methyl-Δ2-1,2,4-thiadiazolin-5-on-hydrazon,
3-Amino-4-methyl-Δ2-1,2,4-thiadiazolin-5-on-hydrazon,
3-Dimethylamino-4-methyl-Δ2-1,2,4-thiadiazolin-5-on-hydrazon,
3-Carboxy-4-methyl-Δ2-1,2,4-thiadiazolin-5-on-hydrazon,
1,4-Dimethyl-Δ2-1,2,4-triazolin-5-on-hydrazon,
1,4-Dihydroxyethyl-Δ2-1,2,4-triazolin-5-on-hydrazon,
1,4-Aminoethyl-Δ2-1,2,4-triazolin-5-on-hydrazon,
1,3,4-Trimethyl-Δ2-1,2,4-triazolin-5-on-hydrazon,
1,4-Dimethyl-3-phenyl-Δ2-1,2,4-triazolin-5-on-hydrazon und
4-Methyl-3-phenyl-Δ2-1,2,4-triazolin-5-on-hydrazon.

Unter den Verbindungen der Formel (I) sind die folgenden Thiazolon-hydrazon-Derivate besonders bevorzugt:
3-Methyl-2(3H)-thiazolon-hyd razon,
3,4-Dimethyl-2(3H)-thiazolon-hydrazon,
4-tert-Butyl-3-methyl-2(3H)-thiazolon-hydrazon,
3-Methyl-4-phenyl-2(3H)-thiazolon-hydrazon,
3-Methyl-4-(4-tolyl)-2(3H)-thiazolon-hydrazon,
4-(4-Methoxy)phenyl-3-methyl-2(3H)-thiazolon-hydrazon,
4-(4-Ethoxy)phenyl-3-methyl-2(3H)-thiazolon-hydrazon,
4-(4-Bromphenyl)-3-methy(-2(3H)-thiazolon-hydrazon,
4-(3-Bromphenyl)-3-methyl-2(3H)-thiazolon-hydrazon,
4-(4-Chlorphenyl)-3-methyl-2(3H)-thiazolon-hydrazon,
4-(3-Chlorphenyl)-3-methyl-2(3H)-thiazolon-hydrazon,
3-Methyl-4-(4-nitrophenyl)-2(3H)-thiazolon-hydrazon,
3-Methyl-4-(3-nitrophenyl)-2(3H)-thiazolon-hydrazon,
4-([1,1'-Biphenyl]-4-yl)-3-methyl-2(3H)-thiazolon-hydrazon,
2-Hydrazono-2,3-dihydro-3-methyl-4-thiazolcarbonsäureethylester,
3,4,5-Trimethyl-2(3H)-thiazolon-hydrazon,
3,4-Dimethyl-5-phenyl-2(3H)-thiazolon-hydrazon,
3,5-Dimethyl-4-phenyl-2(3H)-thiazolon-hydrazon,
3-Methyl-4,5-diphenyl-2(3H)-thiazolon-hydrazon,
5-Ethyl-3-methyl-4-phenyl-2(3H)-thiazolon-hydrazon,
4-(4-Bromphenyl)-3-methyl-5-phenyl-2(3H)-thiazolon-hydrazon,
3-Methyl-5-phenyl-4-(4-tolyl)-2(3H)-thiazolon-hydrazon,
5-(4-Chlorphenyl)-4-phenyl-3-methyl-2(3H)-thiazolon-hydrazon,
5-(4-Chlorphenyl)-4-(4-methoxyphenyl)-3-methyl-2(3H)-thiazolon-hydrazon,
2-Hydrazono-2,3-dihydro-3,4-dimethyl-4-thiazolcarbonsäureethylester,
4-Amino-2-hydrazono-2,3-dihydro-3-methyl-5-thiazolcarbonitril,
3-Ethyl-4,5-dimethyl-2(3H)-thiazolon-hydrazon,
2-Hydrazino-2,3-dihydro-3-ethyl-4-methyl-thiazolcarbonsäureethylester,
5-Methyl-3-(1-methylethyl)-4-phenyl-2(3H)-thiazolon-hydrazon,
4,5-Dimethyl-3-(1-methylethyl)-2(3H)-thiazolon-hydrazon,
3-(1-Methylethyl)-4,5-diphenyl-2(3H)-thiazolon-hydrazon,
4,5-Dimethyl-3-propyl-2(3H)-thiazolon-hydrazon,
4,5-Diphenyl-3-propyl-2(3H)-thiazolon-hydrazon,
3-Butyl-4,5-dimethyl-2(3H)-thiazolon-hydrazon,
3-Butyl-4,5-diphenyl-2(3H)-thiazolon-hydrazon,
4,5-Dimethyl-3-(2-methylpropyl)-2(3H)-thiazolon-hydrazon,
3-(2-Methylpropyl)-4,5-diphenyl-2(3H)-thiazolon-hydrazon,
3-(2-Propenyt)-2(3H)-thiazolon-hydrazon,
4-Methyl-3-(2-propenyl)-2(3H)-thiazolon-hydrazon,
4-tert-Butyl-3-(2-propenyl)-2(3H)-thiazolon-hydrazon,
4-Phenyl-3-(2-propenyl)-2(3H)-thiazolon-hydrazon,
4,5-Dimethyl-3-(2-propenyl)-2(3H)-thiazolon-hydrazon,
4,5-Diphenyl-3-(2-propenyl)-2(3H)-thiazolon-hydrazon,
3-Hydroxyethyl-2(3H)-thiazolon-hydrazon,
3-Hydroxyethyl-4-methyl-2(3H)-thiazolon-hydrazon,
3-Hydroxyethyl-4,5-dimethyl-2(3H)-thiazolon-hydrazon,
3-Aminoethyl-2(3H)-thiazolon-hydrazon,
3-Aminoethyl-4-methyl-2(3H)-thiazolon-hydrazon,
3-Aminoethyl-4,5-dimethyl-2(3H)-thiazolon-hydrazon,
3-Phenyl-2(3H)-thiazolon-hydrazon,
4-Methyl-3-phenyl-2(3H)-thiazolon-hydrazon,
3,4-Diphenyl-2(3H)-thiazolon-hydrazon,
4-p-Biphenylyl-3-phenyl-2(3H)-thiazolon-hydrazon,
4-(4-Methoxy)phenyl-3-phenyol-2(3H)-thiazolon-hydrazon,
4-tert-Butyl-3-phenyl-2(3H)-thiazolon-hydrazon
4,5-Dimethyl-3-phenyl-2(3H)-thiazolon-hydrazon,
5-Methyl-3,4-diphenyl-2(3H)-thiazolon-hydrazon,
3,4,5-Triphenyl-2(3H)-thiazolon-hydrazon,
4,5-Dimethyl-3-(phenylmethyl)-2(3H)-thiazolon-hydrazon,
2-Hydrazono-2,3-dihydro-3-[(phenylamino)carbonyl]-4-methylthiazolcarbonsäureethylester,
3-Methyl-4,5,6,7-tetrahydro-2(3H)-benzothiazolon-hydrazon,
3-Methyl-2(3H)-benzothiazolon-hydrazon,
3,6-Dimethyl-2(3H)-benzothiazolon-hydrazon,
6-Chlor-3-methyl-2(3H)-benzothiazolon-hydrazon,
7-Chlor-3-methyl-2(3H)-benzothiazolon-hydrazon,
6-Hydroxy-3-methyl-2(3H)-benzothiazolon-hydrazon,
5-Methoxy-3-methyl-2(3H)-benzothiazolon-hydrazon,
7-Methoxy-3-methyl-2(3H)-benzothiazolon-hydrazon,
5,6-Dimethoxy-3-methyl-2(3H)-benzothiazolon-hydrazon,
5-Ethoxy-3-methyl-2(3H)-benzothiazolon-hydrazon,
6-Ethoxy-3-methyl-2(3H)-benzothiazolon-hydrazon,
3-Methyl-5-nitro-2(3H)-benzothiazolon-hydrazon,
3-Methyl-6-nitro-2(3H)-benzothiazolon-hydrazon,
5-Acetamido-3-methyl-2(3H)-benzothiazolon-hydrazon,
6-Acetamido-3-methyl-2(3H)-benzothiazolon-hydrazon,
5-Anilino-3-methyl-2(3H)-benzothiazolon-hydrazon,
6-Anilino-3-methyl-2(3H)-benzothiazolon-hydrazon,
2-Hydrazono-2,3-dihydro-3-methyl-6-benzothiazol-carbonsäure,
2-Hydrazono-2,3-dihydro-3-methyl-4-benzothiazol-sulfonsäure,
2-Hydrazono-2,3-dihydro-3-methyl-5-benzothiazol-sulfonsäure,
2-Hydrazono-2,3-dihydro-3-methyl-6-benzothiazol-sulfonsäure,
2-Hydrazono-2,3-dihydro-3-methyl-7-benzothiazol-sulfonsäure,
2-Hydrazono-2,3-dihydro-N,N,3-trimethyl-6-benzothiazol-sulfonsäureamid,
[(2-Hydrazono-2,3-dihydro-3-methyl-6-benzothiazolyl)oxy]essigsäure-hydrazid,
3-Methyl-naphtho[2,3-d]thiazol-2(3H)-on-hydrazon,
3-Ethyl-2(3H)-benzothiazolon-hydrazon,
6-Ethoxy-3-ethyl-2(3H)-benzothiazolon-hydrazon,
3-Propyl-2(3H)-benzothiazolon-hydrazon,
3-Butyl-2(3H)-benzothiazolon-hydrazon,
3-Hexyl-2(3H)-benzothiazolon-hydrazon,
3-Hydroxyethyl-2(3H)-benzothiazolon-hydrazon,
3-Aminoethyl-2(3H)-benzothiazolon-hydrazon,
3-p-Methylbenzyl-2(3H)-benzothiazolon-hydrazon,
2-Hydrazino-2,3-dihydro-3-(2-hydroxyethyl)-6-benzothiazol-carbonsäure,
2-Hydrazono-2,3-dihydro-6-methoxy-3(2H)-benzothiazol-propan-sulfonsäure,
6-Hexadecyloxy-2-hydrazono-3(2H)-benzothiazol-propan-sulfonsäure,
2-Oxo-3-benzothiazolin-essigsäureethylester-hydrazon,
3-Acetyl-2(3H)-benzothiazolon-hydrazon,
2-Hydrazono-3(2H)-benzothiazol-carboxaldehyd.

Die Verbindungen der Formel (I) sind zum Teil im Handel erhältlich. Sie können jedoch auch nach aus der Literatur bekannten Syntheseverfahren, beispielsweise der Vorschrift in Research Disclosure October 1978, Seite 42 - 44, No. 17434, oder in Analogie zu den in der WO 02/074268 bzw. DE 1 049 381 B beschriebenen Verfahren, sowie dem in Journal of Chemical Research, Synopses (1998), Seite 12-13, beschriebenen Verfahren hergestellt werden.

Als Kupplersubstanzen kommen insbesondere die folgenden Kupplersubstanzen oder deren Salze in Betracht:
N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methyl-amino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor 2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Als Persulfatsalze kommen zum Beispiel Kaliumpersulfat, Natriumpersulfat oder Ammoniumpersulfat oder deren Mischungen in Betracht.

Die Persulfatsalze sind in dem gebrauchsfertigen Färbemittel (A) in einer Gesamtmenge von etwa 0,01 bis 10 Gewichtsprozent, vorzugsweise etwa 0,1 bis 5 Gewichtsprozent, enthalten.

Das Wasserstoffperoxid oder dessen Additionsverbindungen sind in dem gebrauchsfertigen Färbemittel (A) in einer Gesamtmenge von etwa 1 bis 10 Gewichtsprozent, vorzugsweise etwa 4 bis 8 Gewichtsprozent, enthalten.

Das Gewichtsverhältnis zwischen Persulfatsalz und Wasserstoffperoxid in dem gebrauchsfertigen Färbemittel (A) beträgt hierbei vorzugsweise etwa 1:1 bis 1:20, insbesondere 1:2 bis 1:10.

Weiterhin kann das erfindungsgemäße Färbemittel zusätzlich zu den Verbindungen der Formel (I) sowie den Kupplersubstanzen gegebenenfalls zusätzlich weitere übliche, physiologisch unbedenkliche, direktziehende Farbstoffe aus der Gruppe der kationischen und anionischen Farbstoffe, der Dispersionsfarbstoffe, der Azofarbstoffe, der Chinonfarbstoffe und der Triphenylmethanfarbstoffe enthalten, sofern diese gegenüber den verwendeten Oxidationsmitteln ausreichend stabil sind.

Die direktziehenden Farbstoffe sind in dem gebrauchsfertigen Färbemittel (A) in einer Menge von etwa 0,01 bis 10 Gewichtsprozent, vorzugsweise etwa 0,1 bis 5 Gewichtsprozent, enthalten.

Weiterhin kann das erfindungsgemäße Färbemittel zusätzlich zu den Verbindungen der Formel (I) gegebenenfalls weitere übliche Entwicklersubstanzen enthalten, sofern diese gegenüber den verwendeten Oxidationsmitteln ausreichend stabil sind

Die Verbindungen der Formel (1) sowie die Kupplersubstanzen und die zusätzlichen Entwicklersubstanzen sind in dem gebrauchsfertigen Färbemittel (A) jeweils in einer Gesamtmenge von etwa 0,01 bis 10 Gewichtsprozent, vorzugsweise etwa 0,1 bis 5 Gewichtsprozent, enthalten.

Die Verbindungen der Formel (I) und die Kupplersubstanzen werden in der Regel getrennt voneinander aufbewahrt und erst kurz vor der Anwendung miteinander vermischt und mit dem Persulfatsalz und dem Wasserstoffperoxid versetzt. Der pH-Wert wird ggfs. anschließend mit einem Alkalisierungsmittel auf den gewünschten alkalischen pH-Wert eingestellt. Es ist jedoch auch möglich, sofern die Verbindungen der Formel (I), die Kupplersubstanzen und das Persulfatsalz sowie ggfs. weitere Zusatzstoffe (beispielsweise direktziehende Farbstoffe und/oder zusätzliche Entwicklersubstanzen) in fester Form vorliegen, diese gemeinsam abzupacken und das gebrauchsfertige Färbemittel (A) kurz vor der Anwendung durch Vermischen der Verbindungen der Formel (I), der Kupplersubstanzen und des Persulfatsalzes sowie der übrigen festen Zusatzstoffe mit der Wasserstoffperoxid-Zubereitung und ggf. einer die übrigen Bestandteile des Mittels enthaltenden flüssigen Zubereitung herzustellen.

Das erfindungsgemäße Färbemittel besteht somit in der Regel aus mehreren Komponenten, welche vor der Anwendung miteinander vermischt werden. Vorzugsweise liegt das Mittel in Form eines Mehrkomponenten-Kits, bestehend aus einer Farbträgermasse (A1), welche die Verbindung der Formel (I) enthält, und einer weiteren Farbträgermasse (A2), welche die Kupplersubstanzen und die Persulfatsalze enthält, und einer das Wasserstoffperoxid und/oder dessen Additionsverbindungen enthaltenden 3. Komponente (A3), sowie ggfs. einem Mittel zur Einstellung des pH-Wertes (Alkalisierungsmittel), vor.

Ein weiterer Gegenstand der vorliegenden Erfindung ist daher ein Mehrkomponenten-Kit, bestehend aus einer Farbträgermasse (A1), welche die Verbindung der Formel (I) enthält, und einer weiteren Farbträgermasse (A2), welche die Kupplersubstanzen und die Persulfatsalze enthält, und einer das Wasserstoffperoxid und/oder dessen Additionsverbindungen enthaltenden 3. Komponente (A3), sowie ggfs. einem Mittel zur Einstellung des pH-Wertes (Alkalisierungsmittel).

Selbstverständlich können auch die vorgenannten Mittel der Komponenten (A1), (A2) und (A3) aus mehreren Einzelkomponenten bestehen, welche erst unmittelbar vor der Anwendung miteinander vermischt werden.

Ebenfalls ist ein Mehrkomponenten-Kit möglich, dessen 1. Komponente aus einem die Verbindungen der Formel (I), die Kupplersubstanzen, die Persulfatsalze und gegebenenfalls das Alkalisierungsmittel sowie weitere übliche pulverförmige kosmetische Zusatzstoffe enthaltenden Pulver besteht, und dessen 2. Komponente eine Wasserstoffperoxid und/oder dessen Additionsverbindungen enthaltende wässrige kosmetische Zubereitung ist.

Als Wasserstoffperoxid kommt eine wässrige Zubereitung (zum Beispiel Lösung oder Emulsion) in Betracht, welche 1 bis 12 Gewichtsprozent, vorzugsweise 6 bis 9 Gewichtsprozent, an Wasserstoffperoxid oder dessen Additionsverbindungen an Harnstoff, Melamin, Natriumborat oder Natriumcarbonat enthält.

Das Gewichtsverhältnis zwischen Farbträgermasse und Wasserstoffperoxidzubereitung beträgt hierbei vorzugsweise etwa 1:1 bis 1:3, insbesondere 1:1 bis 1:2.

Die Verbindungen der Formel (I) sowie die Kupplersubstanzen sind in der jeweiligen Farbträgermasse (Komponente (A1) bzw. Komponente (A2)) jeweils in einer Gesamtmenge von etwa 0,02 bis 20 Gewichtsprozent, vorzugsweise etwa 0,2 bis 10 Gewichtsprozent, enthalten, wobei in dem gebrauchsfertigen Färbemittel (A) die Verbindungen der Formel (1) sowie die Kupplersubstanzen jeweils in einer Gesamtmenge von etwa 0,01 bis 10 Gewichtsprozent, vorzugsweise etwa 0,1 bis 5 Gewichtsprozent, enthalten sind.

Die Zubereitungsform für die Komponenten (A1), (A2) und (A3) sowie des gebrauchsfertigen Färbemittels (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Verbindung der Formel (I) beziehungsweise der Kupplersubstanz und des Oxidationsmittels mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäure-alkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche wecke üblichen Mengen verwendet, zum Beispiel die Netzmittel und Emulgatoren in Konzentrationen von etwa 0,5 bis 30 Gewichtsprozent (jeweils bezogen auf die Komponente (A1) bzw. (A2)), die Verdicker in einer Menge von etwa 0,1 bis 25 Gewichtsprozent (jeweils bezogen auf die Komponente (A1) bzw. (A2)) und die Pflegestoffe in einer Konzentration von etwa 0,1 bis 5,0 Gewichtsprozent (jeweils bezogen auf die Komponente (A1) bzw. (A2)).

Darüber hinaus können in dem Färbemittel noch weitere übliche Zusatzstoffe, beispielsweise Antioxidantien wie Ascorbinsäure, Thioglykolsäure oder Natriumsulfit, sowie Parfümöle, Penetrationsmittel, Puffersysteme, Komplexbildner, Konservierungsstoffe, Netzmittel, Emulgatoren, Verdicker und Pflegestoffe enthalten sein.

Der pH-Wert des gebrauchsfertigen erfindungsgemäßen Färbemittel stellt sich bei der Mischung der Farbträgermasse mit dem Oxidationsmittel auf einen pH-Wert ein, der durch die pH-Werte der Farbträgermasse des Oxidationsmittels sowie durch das Mischungsverhältnis bestimmt wird. Das gebrauchsfertige Mittel (A) weist einen basischen pH-Wert von größer 7, vorzugsweise einen pH-Wert von 8 bis 11, auf. Die basische Einstellung erfolgt hierbei vorzugsweise mit Ammoniak, wobei jedoch auch organische Amine, zum Beispiel 2-Amino-2-methyl-1-propanol, Tris(hydroxymethyl)-amino-methan, Monoethanolamin und Triethanolamin, oder Mischungen von organischen Aminen und Ammoniak sowie anorganische Basen wie Alkalihydroxide, Erdalkalihydroxide, Alkaliacetate, Erdalkaliacetate, Alkalicarbonate oder Erdalkalicarbonate, oder alkanisches Natriumsilikat Verwendung finden können.

Das gebrauchsfertige Färbemittel wird unmittelbar vor der Anwendung durch Vermischen der einzelnen Komponenten (beispielsweise (A1), (A2) und (A3)), ggfs. unter Zusatz eines Alkalisierungsmittel, hergestellt und sodann auf die Faser, insbesondere menschliche Haare, aufgetragen. Je nach gewünschter Farbtiefe läßt man diese Mischung etwa 5 bis 60 Minuten, vorzugsweise etwa 15 bis 30 Minuten, bei einer Temperatur von etwa 20 bis 50 °C, insbesondere bei etwa 30 bis 40 °C einwirken. Anschließend wird die Faser mit Wasser gespült. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird die Keratinfaser getrocknet.

Das erfindungsgemäße Färbemittel ermöglicht eine gleichmässige und dauerhafte Färbung von Keratinfasern, insbesondere menschlichen Haaren, die das komplette Farbspektrum abdecken und sich insbesondere durch ihre besondere Farbintensität und Leuchtkraft, auszeichnen.

Die nachfolgenden Beispiele sollen den Gegenstand näher erläutern, ohne ihn auf diese Beispiele zu beschränken.

### Beispiele

### Beispiel 1a: Synthese von 3,4-Dimethyl-2(3H)-thiazolon-hydrazon-Hydrochlorid

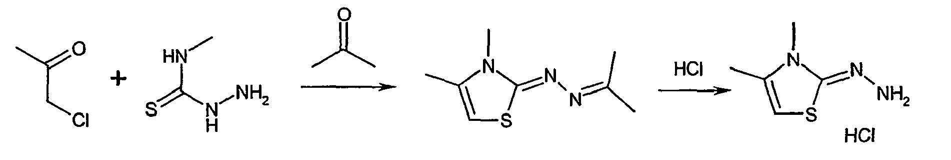

### Stufe A: 3,4-Dimethyl-2(3H)-thiazolon-(1-methylethyliden)hydrazon

21 g (200 mmol) 4-Methyl-3-thiosemicarbazid werden in 1000 ml Aceton 2 Stunden lang unter Rückfluss gekocht. Dann wird die Lösung tropfenweise mit 20,4 g (220 mmol) Chloraceton versetzt. Die Reaktionsmischung wird sodann 7 Stunden lang unter Rückfluss gekocht, und anschließend eingeengt. Das so erhaltene Rohprodukt wird aus Aceton umkristallisiert. Es werden 23 g eines orangen Pulvers (63% der Theorie) erhalten.
Schmelzpunkt: 139 -139,6 °C
¹H-NMR (DMSO, 300 MHz): δ = 6,72 (s, breit, 1H, H-C(5)); δ = 3,67 (s, 3H, N-CH3); δ = 2,27 (d, J=0,9Hz, 3H, CH3-C(4)); δ = 2,17 (s, 3H, CH3); δ = 2,07 (s, 3H, CH3)
¹³C-NMR (DMSO, 300 MHz): δ = 169,16; δ = 164,14; δ = 139,02 (C(4)); δ = 103,36 (C(5)); δ = 34,47 (CH₃N); δ = 24,60; δ = 19,91; δ = 13,53 (CH₃-C(4)).
MS (ESI): 184 (M⁺ +1)

### Stufe B: 3,4-Dimethyl-2(3H)-thiazolon-hydrazon-Hydrochlorid

3,5 g (19 mmol) 3,4-Dimethyl-2(3H)-thiazolon-(1-methylethyliden)-hydrazon aus Stufe A werden in 60 ml 6M Salzsäure bei 50 °C 30 Minuten lang erwärmt. Die Reaktionsmischung wird anschließend eingeengt und das Rohprodukt wird sodann aus Ethanol umkristallisiert. Es werden 2 g (60% der Theorie) eines rosa Pulvers erhalten.
Schmelzpunkt: 156,4 - 156,6 °C
¹H-NMR (DMSO. 300 MHz): δ = 6,58 (q, J=0,9 Hz, 1H, H-C(5)); δ = 3,41 (s, 3H, N-CH3); δ = 2,18 (d, J=0,9Hz, 3H, CH3-C(4)).
MS (ESI): 144 (M⁺ +1).
¹³ C-NMR (DMSO. 300 MHz): δ = 172,30 (C(2)); δ = 138,79 (C(4)); δ = 101,43 (C(5)); δ = 32,92 (CH₃N); δ = 13,40 (CH₃-(C4)).
CHN-Analyse:

| (C₅H₉N₃S (0,96 HCl) (0,5 EtOH)): | | | | | |
|---|---|---|---|---|---|
| | % C | % H | % N | % S | % Cl |
| berechnet: | 35,81 | 6,49 | 20,88 | 15,93 | 16,90 |
| gefunden: | 35,20 | 6,30 | 21,00 | 15,40 | 16,80 |

### Beispiele 1b-1g: Synthese von 2(3H)-Thiazolon-hydrazonen der Formel (I) (allgemeine Synthese Vorschrift)

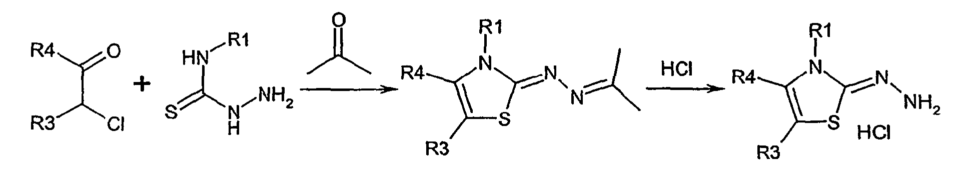

**Stufe A:** 4 mmol substituiertes Thiosemicarbazid werden in 20 ml Aceton 2 Stunden lang unter Rückfluss gekocht. Dann wird die Lösung tropfenweise mit 4,4 mmol α-Chlor-keton versetzt. Die Reaktionsmischung wird sodann 7 Stunden lang unter Rückfluss gekocht, und anschließend eingeengt. Das so erhaltene 2(3H)-Thiazolon-1-(methylethyliden)-hydrazon Derivat wird aus Aceton umkristallisiert.
**Stufe B:** 2 mmol des 2(3H)-Thiazolon-1-(methylethyliden)-hydrazon Derivates aus Stufe A werden in 10 ml 6M Salzsäure bei 50 °C 30 Minuten lang erwärmt. Die Reaktionsmischung wird anschließend eingeengt und das Rohprodukt wird sodann aus Ethanol oder Butanol umkristallisiert.

### 1b.) 3-Methyl-4-phenyl-2(3H)-thiazolon-hydrazon Hydrochlorid

Aus 4-Methyl-3-thiosemicarbazid und Phenacylchlorid
¹H-NMR (DMSO/D₂O, 300 MHz): δ = 7,49-7,42 (m, 5H, phenyl); δ = 6,84 (s, 1H, H-C(5)); δ = 3,31 (s, 3H, N-CH₃).
ESI-MS: 205 [M]⁺ (100)

### 1c.) 4-tert-Butyl-3-methyl-2(3H)-thiazolon-hydrazon Hydrochlorid

Aus 4-Methyl-3-thiosemicarbazid und 1-Chlor-3,3-dimethy(-2-butanon
¹H-NMR (DMSO/D₂O, 300 MHz): δ = 6,55 (s, 1H, H-C(5)); δ = 3,60 (s, 3H N-CH₃); δ = 1,31 (s, 9H, (CH₃)₃).
ESI-MS: 185 [M]⁺ (100)

### 1d.) 4-Methyl-3-(2-propenyl)- 2(3H)-thiazolon-hydrazon Hydrochlorid

Aus 4-(2-propenyl)-3-thiosemicarbazid und Chloraceton
¹H-NMR (DMSO/D₂O, 300 MHz): δ = 6,58 (s, 1H, H-C(5)); δ = 5,94-5,81 (m, 1H, Allyl); δ = 5,22 (dd, 1H, J=0,9 Hz, J=10,5 Hz, Allyl); δ = 4,94 (dd, 1H, J=0,9 Hz, J=17,1 Hz, Allyl); δ = 4,57 (m, 2H, N-CH₂); δ = 2,16 (s, 3H, CH₃-C(4)).
ESI-MS: 169 [M]⁺ (100)

### 1e.) 4-Phenyl-3-(2-propenyl)- 2(3H)-thiazolon-hydrazon Hydrochlorid

Aus 4-(2-propenyl)-3-thiosemicarbazid und Phenacylchlorid
¹H-NMR (DMSO/D₂O, 300 MHz): δ = 7,50-7,42 (m, 5H, phenyl); δ = 6,81 (s, 1H, H-C(5)); δ = 5,77-5,63 (m, 1H, Allyl); δ = 5,15 (dd, 1H, J=0,9 Hz, J=10,5 Hz, Allyl); δ = 4,80 (dd, 1H, J=0,9 Hz, J=17,1 Hz, Allyl); δ = 4,40 (m, 2H, N-CH₂); δ = 1,27 (s, 9H, CH₃-C(4)).
ESI-MS: 231 [M]⁺ (100)

### 1f.) 4-tert-Butyl-3-(2-propenyl)-2(3H)-thiazolon-hydrazon Hydrochlorid

Aus 4-(2-propenyl)-3-thiosemicarbazid und 1-Chlor-3,3-dimethyl-2-butanon
¹H-NMR (DMSO/D₂O, 300 MHz): δ = 6,55 (s, 1H, H-C(5)); δ = 5,90-5,77 (m, 1H, Allyl); δ = 5,21 (d, 1H, J=9,0 Hz, Allyl); δ = 4,81-4,75 (m, 3H, Allyl); δ = 1,31 (s, 9H, (CH₃)₃).
ESI-MS: 211 [M]⁺ (100)

### 1g.) 3,4,5-Trimethyl-2(3H)-thiazolon-hydrazon Hydrochlorid

Aus 4-Methyl-3-thiosemicarbazid und 3-Chlor-2-butanon
¹H-NMR (DMSO/D₂O, 300 MHz): δ = 3,55 (s, 3H, N-CH₃); δ = 2,16 (s, 3H, CH₃; δ = 2,12 (s, 3H, CH₃).
ESI-MS: 157 [M]⁺ (100)

### Beispiele 2-9: Färbemittel mit 3-Methyl-2(3H)-benzothiazolon-hydrazon-Hydrochlorid

### Komponente (A1)

| | |
|---|---|
| 4,00 g | Decylpolyglucose (Plantaren® 2000), wässrige Lösung |
| 0,20 g | Ethylendiaminotetraessigsäure-Dinatriumsalz-Hydrat |
| 0,50 g | Ethanol |
| 0,58 g | 3-Methyl-2(3H)-benzothiazolon-hydrazon-Hydrochlorid-Hydrat |
| ad 100,0 g | Wasser, entmineralisiert |

### Komponente (A2)

| | |
|---|---|
| Y g | Kuppler gemäß Tabelle 1 |
| 0,67 g | Kaliumpersulfat |

Bei Raumtemperatur (20-25 °C) oder unter leichtem Erwärmen (35-40 °C) werden 3,3 g der Komponente (A1) mit Komponente (A2) versetzt und dann mit 6,6 g einer 9%igen Wasserstoffperoxidlösung vermischt. Der pH-Wert des gebrauchsfertigen Färbemittels (A) wird mit einer 25%igen wässrigen Ammoniaklösung auf 9,5 eingestellt. Das gebrauchsfertige Haarfärbemittel wird auf Naturhaarsträhnen aufgetragen und mit einem Pinsel gleichmäßig verteilt. Nach einer Einwirkungszeit von 30 Minuten bei 40 °C wird das Haar mit einem handelsüblichen Shampoo gewaschen, mit lauwarmem Wasser gespült und sodann getrocknet.

Die Einsatzmenge der Kuppler sowie die erhaltenen Färbungen sind in der nachfolgenden Tabelle 1 zusammengefaßt.

**Tabelle 1:**

| **Bsp. Nr.** | **Verwendetes Amin bzw. Phenol** | **Farbton** |
|---|---|---|
| | **(Menge in g)** | **nach der Färbung** |
| **2** | 1,3-Diamino-benzol (0,27 g) | rubinrot |
| **3** | 2,4-Diamino-1-(2-hydroxyethoxy)benzol-sulfat (0,66 g) | mahagoni |
| **4** | 2-Amino-4-[(2-hydroxyethyl)amino]anisol-sulfat (0,74 g) | mahagoni |
| **5** | N-(3-Dimethylamino-phenyl)-harnstoff (0,44 g) | dunkel-violett |
| **6** | 3-Aminophenol (0,27 g) | kupferrot |
| **7** | 5-Amino-2-methyl-phenol (0,31 g) | goldgelb |
| **8** | 1,3-Dihydroxybenzol (0,27 g) | gelborange |
| **9** | 1-Naphthol (0,36 g) | orangerot |

### Beispiele 10-17: Färbemittel mit 3,4-Dimethyl-2(3H)-thiazolon-hydrazon-Hydrochlorid

### Komponente (A1)

| | |
|---|---|
| 4,00 g | Decylpolyglucose (Plantaren® 2000), wässrige Lösung |
| 0,20 g | Ethylendiaminotetraessigsäure-Dinatriumsalz-Hydrat |
| 5,00 g | Ethanol |
| 0,45 g | 3,4-Dimethyl-2(3H)-thiazolon-hydrazon-Hydrochlorid |
| ad 100,00 g | Wasser, entmineralisiert |

### Komponente (A2)

| | |
|---|---|
| Y g | Kuppler gemäß Tabelle 2 |
| 0,67 g | Kaliumpersulfat |

Bei Raumtemperatur (20-25 °C) oder unter leichtem Erwärmen (35-40 °C) werden 3,3 g der Komponente (A1) mit Komponente (A2) versetzt und dann mit 6,6 g einer 9%igen Wasserstoffperoxidlösung vermischt. Der pH-Wert des gebrauchsfertigen Färbemittels (A) wird mit einer 25%igen wässrigen Ammoniak auf 10 eingestellt. Das gebrauchsfertige Haarfärbemittel wird auf Naturhaarsträhnen aufgetragen und mit einem Pinsel gleichmäßig verteilt. Nach einer Einwirkungszeit von 30 Minuten bei 40 °C wird das Haar mit einem handelsüblichen Shampoo gewaschen, mit lauwarmem Wasser gespült und sodann getrocknet.

Die Einsatzmenge der Kuppler sowie die erhaltenen Färbungen sind in der nachfolgenden Tabelle 2 zusammengefaßt.

**Tabelle 2:**

| **Bsp. Nr.** | **Verwendetes Amin bzw. Phenol** | **Farbton nach der Färbung** |
|---|---|---|
| | **(Menge in g)** | |
| **10.** | 1,3-Diamino-benzol (0,27 g) | bordeauxrot |
| **11.** | 2,4-Diamino-1-(2-hydroxyethoxy)benzol-sulfat (0,67 g) | dunkel bordeauxrot |
| **12.** | 2-Amino-4-[(2-hydroxyethyl)amino]anisol-sulfat (0,75 g) | dunkel bordeauxrot |
| **13.** | N-(3-Dimethylamino-phenyl)-harnstoff (0,44 g) | blau |
| **14.** | 3-Aminophenol (0,27 g) | himbeerrot |
| **15.** | 5-Amino-2-methyl-phenol (0,31 g) | kupferrot |
| **16.** | 1,3-Dihydroxybenzol (0,27 g) | kupferfarben |
| **17.** | 1-Naphthol (0,36 g) | rosa |

### Beispiele 18-23: Färbemittel mit 2(3H)-Thiazolon-hydrazon der Formel (I)

### Komponente (A1)

| | |
|---|---|
| 4,00 g | Decylpolyglucose (Plantaren® 2000), wässrige Lösung |
| 0,20 g | Ethylendiaminotetraessigsäure-Dinatriumsalz-hlydrat |
| 5,00 g | Ethanol |
| 0,45 g | 3,4-Dimethyl-2(3H)-thiazolon-hydrazon-Hydrochlorid |
| ad 100,00 g | Wasser, entmineralisiert |

### Komponente (A2)

| | |
|---|---|
| Y g | Kuppler gemäß Tabelle 2 |
| 0,67 g | Kaliumpersulfat |

Bei Raumtemperatur (20-25 °C) oder unter leichtem Erwärmen (35-40 °C) werden 3,3 g der Komponente (A1) mit Komponente (A2) versetzt und dann mit 6,6 g einer 9%igen Wasserstoffperoxidlösung vermischt. Der pH-Wert des gebrauchsfertigen Färbemittels (A) wird mit einer 25%igen wässrigen Ammoniak auf 10 eingestellt. Das gebrauchsfertige Haarfärbemittel wird auf Naturhaarsträhnen aufgetragen und mit einem Pinsel gleichmäßig verteilt. Nach einer Einwirkungszeit von 30 Minuten bei 40 °C wird das Haar mit einem handelsüblichen Shampoo gewaschen, mit lauwarmem Wasser gespült und sodann getrocknet.

Die Einsatzmenge der Kuppler sowie die erhaltenen Färbungen sind in der nachfolgenden Tabelle 3 zusammengefaßt.

| **Bsp. Nr** | **Derivat der Formel (I) gemäss Beispiel Nr (Menge in g)** | **Verwendete Kupplersubstanz (Menge in g)** | **Farbton** |
|---|---|---|---|
| **18** | Beispiel 1b | 3-Aminophenol | himbeerrot |
| | (0,60 g) | (0,27 g) | |
| **19** | Beispiel 1c | 3-Aminophenol | himbeerrot |
| | (0,55 g) | (0,27 g) | |
| **20** | Beispiel 1g | 3-Aminophenol | bordeauxrot |
| | (0,48 g) | (0,27 g) | |
| **21** | Beispiel 1b | 5-Amino-2-methyl- | kupferrot |
| | (0,60 g) | phenol (0,31 g) | |
| **22** | Beispiel 1c | 5-Amino-2-methyl- | kupferrot |
| | (0,55 g) | phenol (0,31 g) | |
| **23** | Beispiel 1g | 5-Amino-2-methyl- | kupferrot |
| | (0,48 g) | phenol (0,31 g) | |

Alle Prozentangaben in der vorliegenden Anmeldung stellen, sofern nicht anders angegeben, Gewichtsprozente dar.

## Patentansprüche

1. Gebrauchsfertiges Mittel zum gleichzeitigen Aufhellen und Färben von Keratinfasern (A) auf der Basis einer Entwicklersubstanz-Kupplersubstanz-Kombination, weiches einen basischen pH-Wert aufweist, **dadurch gekennzeichnet, dass** es (a) mindestens ein heterozyklisches Hydrazon-Derivat der Formel (I) oder dessen physiologisch verträgliches Salz, 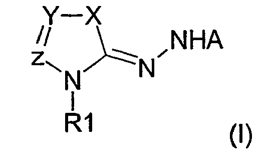 worin
**X** gleich Sauerstoff, Schwefel oder N-R2 ist,
**Y** gleich C-R3 oder Stickstoff ist und
**Z** gleich C-R4 oder Stickstoff ist,
mit der Bedingung, dass der heterozyklische Teil der Verbindung der Formel (1) maximal drei Heteroatome enthält;
**A** Wasserstoff, eine Acetylgruppe, eine Trifluoracetylgruppe, eine Formylgruppe, eine (C₁-C₆)-Alkylsulfonylgruppe oder eine Arylsulfonylgruppe darstellt;
**R1** und **R2** gleich oder verschieden sein können, und unabhängig voneinander eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine Amino-(C₁-C₁₂)-alkylgruppe, eine Sulfonsäure-(C₁-C₁₂)-alkylgruppe, eine Formylgruppe, einer C(O)-(C₁-C₁₂)-Alkylgruppe, eine C(O)-Phenylgruppe, eine C(O)NH-Alkylgruppe, eine C(O)NH-Phenylgruppe, eine substituierte oder unsubstituierte Phenylgruppe oder eine Benzylgruppe darstellt;
**R3** und **R4** gleich oder verschieden sein können und unabhängig voneinander Wasserstoff, ein Halogenatom, eine gesättigte oder ungesättigte (C₁-C₁₂)-Alkylgruppe, eine mit einem Halogenatom substituierte (C₁-C₁₂)-Alkylgruppe, eine Hydroxygruppe, eine Hydroxy-(C₁-C₁₂)-alkylgruppe, eine (C₁-C₁₂)-Alkoxygruppe, eine Cyanogruppe, eine Nitrogruppe, eine Aminogruppe, eine (C₁-C₁₂)-Alkylaminogruppe, eine (C₁-C₁₂)-Dialkylaminogruppe, eine Carbonsäure, eine C(O)O-(C₁-C₁₂)-Alkylgruppe, eine substituierte oder unsubstituierte C(O)O-Phenylgruppe, eine substituierte oder unsubstituierte Phenylgruppe oder eine Naphthylgruppe darstellen;
und wenn **Y** und **Z** gleich C-R3 und C-R4 sind, **R3** und **R4** gemeinsam mit dem Restmolekül ein heterozyklisches oder carbozyklisches, gesättigtes oder ungesättigtes, substituiertes oder unsubstituiertes Ringsystem bilden;
(b) mindestens eine an sich bekannte Kupplersubstanz oder deren physiologisch verträgliches Salz, und
(c) als Oxidationsmittel eine Kombination aus mindestens einem Persulfatsalz und Wasserstoffperoxid und/oder dessen Additionsverbindungen enthält.

2. Mittel nach Anspruch 1, **dadurch gekennzeichnet, dass** (i) **X** gleich Schwefel ist, **Y** gleich C-R3 ist, **Z** gleich C-R4 ist und **A** ein Wasserstoffatom darstellt, oder (ii) **X** gleich N-R2 ist, **Y** gleich Stickstoff ist und **A** ein Wasserstoffatom darstellt.

3. Mittel nach Anspruch 1 oder 2, **dadurch gekennzeichnet, dass** das Hydrazon-Derivat der Formel (I) ausgewählt ist 3-Methyl-2(3H)-thiazolon-hydrazon, 3,4-Dimethyl-2(3H)-thiazolon-hydrazon, 4-tert-Butyl-3-methyl-2(3H)-thiazolon-hydrazon, 3-Methyl-4-phenyl-2(3H)-thiazolon-hydrazon, 3-Methyl-4-(4-tolyl)-2(3H)-thiazolon-hydrazon, 4-(4-Methoxy)phenyl-3-methyl-2(3H)-thiazolon-hydrazon, 4-(4-Ethoxy)phenyl-3-methyl-2(3H)-thiazolon-hydrazon, 4-(4-Bromphenyl)-3-methyl-2(3H)-thiazolon-hydrazon, 4-(3-Bromphenyl)-3-methyl-2(3H)-thiazolon-hydrazon, 4-(4-Chlorphenyl)-3-methyl-2(3H)-thiazolon-hydrazon, 4-(3-Chlorphenyl)-3-methyl-2(3H)-thiazolon-hydrazon, 3-Methyl-4-(4-nitrophenyl)-2(3H)-thiazolon-hydrazon, 3-Methyl-4-(3-nitrophenyl)-2(3H)-thiazolon-hydrazon, 4-([1,1'-Biphenyl]-4-yl)-3-methyl-2(3H)-thiazolon-hydrazon, 3-Methyl-4-(2-naphthalenyl)-2(3H)-thiazolon-hydrazon, 2-Hydrazono-2,3-dihydro-3-methyl-4-thiazol-carbonsäureethylester, 3,4,5-Trimethyl-2(3H)-thiazolon-hydrazon, 3,4-Dimethyl-5-phenyl-2(3H)-thiazolon-hydrazon, 3,5-Dimethyl-4-phenyl-2(3H)-thiazolon-hydrazon, 3-Methyl-4,5-diphenyl-2(3H)-thiazolon-hydrazon, 5-Ethyl-3-methyl-4-phenyl-2(3H)-thiazolon-hydrazon, 4-(4-Bromphenyl)-3-methyl-5-phenyl-2(3H)-thiazolon-hydrazon, 3-Methyl-5-phenyl-4-(4-tolyl)-2(3H)-thiazolon-hydrazon, 5-(4-Chlorphenyl)-4-phenyl-3-methyl-2(3H)-thiazolon-hydrazon, 5-(4-Chlorphenyl)-4-(4-methoxyphenyl)-3-methyl-2(3H)-thiazolon-hydrazon, 2-Hydrazono-2,3-dihydro-3,4-dimethyl-4-thiazolcarbonsäureethylester, 4-Amino-2-hydrazono-2,3-dihydro-3-methyl-5-thiazolcarbonitril, 3-Ethyl-4,5-dimethyl-2(3H)-thiazolon-hydrazon, 2-Hydrazono-2,3-dihydro-3-ethyl-4-methyl-thiazolcarbonsäureethylester, 5-Methyl-3-(1-methylethyl)-4-phenyl-2(3H)-thiazolon-hydrazon, 4,5-Dimethyl-3-(1-methylethyl)-4-phenyl-2(3H)-thiazolon-hydrazon, 3-(1-Methylethyl)-4,5-diphenyl-2(3H)-thiazolon-hydrazon, 4,5-Dimethyl-3-propyl-2(3H)-thiazolon-hydrazon, 4,5-Diphenyl-3-propyl-2(3H)-thiazolon-hydrazon, 3-Butyl-4,5-dimethyl-2(3H)-thiazolon-hydrazon, 3-Butyl-4,5-diphenyl-2(3H)-thiazolon-hydrazon, 4,5-Dimethyl-3-(2-methylpropyl)-2(3H)-thiazolon-hydrazon, 3-(2-Methylpropyl)-4,5-diphenyl-2(3H)-thiazolon-hydrazon, 3-Hydroxyethyl-2(3H)-thiazolon-hydrazon, 3-Hydroxyethyl-4-methyl-2(3H)-thiazolon-hydrazon, 3-Hydroxyethyl-4,5-dimethyl-2(3H)-thiazolon-hydrazon, 3-Aminoethyl-2(3H)-thiazolon-hydrazon, 3-Aminoethyl-4-methyl-2(3H)-thiazolon-hydrazon, 3-Aminoethyl-4,5-dimethyl-2(3H)-thiazolon-hydrazon, 3,4-Diphenyl-2(3H)-thiazolon-hydrazon, 4-Methyl-3-phenyl-2(3H)-thiazolon-hydrazon, 4-p-Biphenylyl-3-phenyl-2(3H)-thiazolon-hydrazon, 4-(4-Methoxy)phenyl-3-phenyl-2(3H)-thiazolon-hydrazon, 4-tert-Butyl-3-phenyl-2(3H)-thiazolon-hydrazon, 4,5-Dimethyl-3-phenyl-2(3H)-thiazolon-hydrazon, 5-Methyl-3,4-diphenyl-2(3H)-thiazolon-hydrazon, 3,4,5-Triphenyl-2(3H)-thiazolon-hydrazon, 4,5-Dimethyl-3-(phenylmethyl)-2(3H)-thiazolon-hydrazon, 3-(2-Propenyl)-2(3H)-thiazolon-hydrazon, 4-Methyl-3-(2-propenyl)-2(3H)-thiazolon-hydrazon, 4-tert-Butyl-3-(2-propenyl)-2(3H)-thiazolon-hydrazon, 4-Phenyl-3-(2-propenyl)-2(3H)-thiazolon-hydrazon, 4,5-Dimethyl-3-(2-propenyl)-2(3H)-thiazolon-hydrazon, 4,5-Diphenyl-3-(2-propenyl)-2(3H)-thiazolon-hydrazon, 4,5-Dimethyl-3-(phenylmethyl)-2(3H)-thiazolon-hydrazon, 2-Hydrazono-2,3-dihydro-3-[(phenylamino)carbonyl]-4-methyl-thiazolcarbonsäurethylester, 3-Methyl-4,5,6,7-tetrahydro-2(3H)-benzothiazolon-hydrazon, 3-Methyl-2(3H)-benzothiazolon-hydrazon, 3,6-Dimethyl-2(3H)-benzothiazolon-hydrazon, 6-Chlor-3-methyl-2(3H)-benzothiazolon-hydrazon, 7-Chlor-3-methyl-2(3H)-benzothiazolon-hydrazon, 6-Hydroxy-3-methyl-2(3H)-benzothiazolon-hydrazon, 5-Methoxy-3-methyl-2(3H)-benzothiazolon-hydrazon, 7-Methoxy-3-methyl-2(3H)-benzothiazolon-hydrazon, 5,6-Dimethoxy-3-methyl-2(3H)-benzothiazolon-hydrazon, 5-Ethoxy-3-methyl-2(3H)-benzothiazolon-hydrazon, 6-Ethoxy-3-methyl-2(3H)-benzothiazolon-hydrazon, 3-Methyl-5-nitro-2(3H)-benzothiazolon-hydrazon, 3-Methyl-6-nitro-2(3H)-benzothiazolon-hydrazon, 5-Acetamido-3-methyl-2(3H)-benzothiazolon-hydrazon, 6-Acetamido-3-methyl-2(3H)-benzothiazolon-hydrazon, 5-Anilino-3-methyl-2(3H)-benzothiazolon-hydrazon, 6-Anilino-3-methyl-2(3H)-benzothiazolon-hydrazon, 2-Hydrazono-2,3-dihydro-3-methyl-6-benzothiazol-carbonsäure, 2-Hydrazono-2,3-dihydro-3-methyl-4-benzothiazol-sulfonsäure, 2-Hydrazono-2,3-dihydro-3-methyl-5-benzothiazol-sulfonsäure, 2-Hydrazono-2,3-dihydro-3-methyl-6-benzothiazol-sulfonsäure, 2-Hydrazono-2,3-dihydro-3-methyl-7-benzothiazol-sulfonsäure, 2-Hydrazono-2,3-dihydro-N,N,3-trimethyl-6-benzothiazol-sulfonsäureamid, [(2-Hydrazono-2,3-dihydro-3-methyl-6-benzothiazolyl)oxy]essigsäurehydrazid, 3-Methyl-naphtho[2,3-d]thiazol-2(3H)-on-hydrazon, 3-Ethyl-2(3H)-benzothiazolon-hydrazon, 6-Ethoxy-3-ethyl-2(3H)-benzothiazolon-hydrazon, 3-Propyl-2(3H)-benzothiazolon-hydrazon, 3-Butyl-2(3H)-benzothiazolon-hydrazon, 3-Hexyl-2(3H)-benzothiazolon-hydrazon, 3-Hydroxyethyl-2(3H)-benzothiazolon-hydrazon, 3-Aminoethyl-2(3H)-benzothiazolon-hydrazon, 3-p-Methylbenzyl-2(3H)-benzothiazolon-hydrazon, 2-Hydrazono-2,3-dihydro-3-(2-hydroxyethyl)-6-benzothiazol-carbonsäure, 2-Hydrazono-2,3-dihydro-6-methoxy-3(2H)-benzothiazol-propan-sulfonsäure, 6-Hexadecyloxy-2-hydrazono-3(2H)-benzothiazol-propan-sulfonsäure, 2-Oxo-3-benzothiazolin-essigsäureethylester-hydrazon, 3-Acetyl-2(3H)-benzothiazolon-hydrazon, 2-Hydrazono-3(2H)-benzothiazol-carboxaldehyd, 3-Methyl-2(3H)-oxazolon-hydrazon, 3-Phenyl-2(3H)-oxazolon-hydrazon, 3-Methyl-2(3H)-benzoxazolon-hydrazon, 3-Phenyl-2(3H)-benzoxazolon-hydrazon, 1,3-Dimethyl-4-imidazolin-2-on-hydrazon, 1,3-Diethyl-4-imidazolin-2-on-hydrazon, 1,3-Dihydroxyethyl-4-imidazolin-2-on-hydrazon, 1,3-Diaminoethyl-4-imidazolin-2-on-hydrazon, 1,3-Dimethyl-4-methoxy-4-imidazolin-2-on-hydrazon, 1,3,4-Trimethyl-4-imidazolin-2-on-hydrazon, 1,3-Dimethyl-4-phenyl-4-imidazolin-2-on-hydrazon, 4-Carboxy-1,3-dimethyl-4-imidazolin-2-on-hydrazon, 4-Amino-1,3-dimethyl-4-imidazolin-2-on-hydrazon, 1,3-Dimethyl-4-dimethylamino-4-imidazolin-2-on-hydrazon, 1,3-Dimethyl-2-benzimidazofinon-hydrazon, 1,3-Diethyl-2-benzimidazolinon-hydrazon, 1,3-Dihydroxyethyl-2-benzimidazolinon-hydrazon, 1,3-Diaminoethyl-2-benzimidazolinon-hydrazon, 1,3,5-Trimethyl-2-benzimidazolinon-hydrazon, 5-Methoxy-1,3-dimethyl-2-benzimidazolinon-hydrazon, 5-Brom-1,3-dimethyl-2-benzimidazolinon-hydrazon, 4,6-Dibrom-1,3-dimethyl-2-benzimidazolinon-hydrazon, 5-Chlor-1,3-dimethyl-2-benzimidazolinon-hydrazon, 1,3-Dimethyl-5-nitro-2-benzimidazolinon-hydrazon, 1,3-Dimethyl-6-nitro-2-benzimidazolinon-hydrazon, 1,4-Dimethyl-Δ2-1,2,4-triazolin-5-on-hydrazon, 1,4-Dihydroxyethyl-Δ2-1,2,4-triazolin-5-on-hydrazon, 1,4-Diaminoethyl-Δ2-1,2,4-triazolin-5-on-hydrazon, 1,3,4-Trimethyl-Δ2-1,2,4-triazolin-5-on-hydrazon, 1,4-Dimethyl-3-phenyl-Δ2-1,2,4-triazolin-5-on-hydrazon, 1,4-Dimethyl-3-methoxy-Δ2-1,2,4-triazolin-5-on-hydrazon, 1,4-Dimethyl-3-dimethylamino-Δ2-1,2,4-triazolin-5-on-hydrazon, 4-Carboxy-1,4-dimethyl-Δ2-1,2,4-triazolin-5-on-hydrazon, 4-Amino-1,4-dimethyl-Δ2-1,2,4-triazolin-5-on-hydrazon, 4-Butyl-1-methyl-3-phenyl-Δ2-1,3,4-triazolin-5-on-hydrazon, 4-Methyl-Δ2-1,3,4-thiadiazolin-5-on-hydrazon, 4-Hydroxyethyl-Δ2-1,3,4-thiadiazolin-5-on-hydrazon, 4-Aminoethyl-Δ2-1,3,4-thiadiazolin-5-on-hydrazon, 4-Methyl-2-phenyl-Δ2-1,3,4-thiadiazolin-5-on-hydrazon, 2-Methoxy-4-methyl-Δ2-1,3,4-thiadiazolin-5-on-hydrazon, 2-Anilino-4-methyl-Δ2-1,3,4-thiadiazolin-5-on-hydrazon, 2-Amino-4-methyl-Δ2-1,3,4-thiadiazolin-5-on-hydrazon, 2-Dimethylamino-4-methyl-Δ2-1,3,4-thiadiazolin-5-on-hydrazon, 4-Methyl-2-(methylthio)-Δ2-1,3,4-thiadiazolin-5-on-hydrazon, 4-(5-Hydrazono-4,5-dihydro-4-methyl-1,3,4-thiadiazol-2-yl)-benzensulfonylfluorid, 4-Methyl-Δ2-1,2,4-thiadiazolin-5-on-hydrazon, 4-Hydroxyethyl-Δ2-1,2,4-thiadiazolin-5-on-hydrazon, 4-Aminoethyl-Δ2-1,2,4-thiadiazolin-5-on-hydrazon, 4-Methyl-3-phenyl-Δ2-1,2,4-thiadiazolin-5-on-hydrazon, 3-Methoxy-4-methyl-Δ2-1,2,4-thiadiazolin-5-on-hydrazon, 3-Amino-4-methyl-Δ2-1,2,4-thiadiazolin-5-on-hydrazon, 3-Dimethylamino-4-methyl-Δ2-1,2,4-thiadiazolin-5-on-hydrazon, 3-Carboxy-4-methyl-Δ2-1,2,4-thiadiazolin-5-on-hydrazon, 1,4-Dimethyl-Δ2-1,2,4-triazolin-5-on-hydrazon, 1,4-Dihydroxyethyl-Δ2-1,2,4-triazolin-5-on-hydrazon, 1,4-Aminoethyl-Δ2-1,2,4-triazolin-5-on-hydrazon, 1,3,4-Trimethyl-Δ2-1,2,4-triazolin-5-on-hydrazon, 1,4-Dimethyl-3-phenyl-Δ2-1,2,4-triazolin-5-on-hydrazon und 4-Methyl-3-phenyl-Δ2-1,2,4-triazolin-5-on-hydrazon.

4. Mittel nach einem der Ansprüche 1 bis 3, **dadurch gekennzeichnet, dass** die Kupplersubstanz ausgewählt ist aus N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxy-ethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methyl-amino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxy-propoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methylbenzol, 3,4-Methylendioxy-phenol, 3,4-Methylen-dioxy-anilin, 5-[(2-Hydroxyethyl)amino]-1,3-benzo-dioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

5. Mittel nach einem der Ansprüche 1 bis 4, **dadurch gekennzeichnet, dass** das Persulfatsalz ausgewählt ist aus Kaliumpersulfat, Natriumpersulfat und Ammoniumpersulfat.

6. Mittel nach einem der Ansprüche 1 bis 5, **dadurch gekennzeichnet, dass** es die Hydrazon-Derivate der Formel (I), sowie die Kupplersubstanzen und die Persulfatsalze jeweils in einer Gesamtmenge von 0,01 bis 10 Gewichtsprozent enthält.

7. Mittel nach einem der Ansprüche 1 bis 6, **dadurch gekennzeichnet, dass** es zusätzlich 0,01 bis 10 Gewichtsprozent eines physiologisch unbedenklichen, direktziehenden Farbstoffs enthält.

8. Mittel nach einem der Ansprüche 1 bis 7, **dadurch gekennzeichnet, dass** es einen pH-Wert von 7 bis 10 aufweist.

9. Mittel nach einem der Ansprüche 1 bis 8, **dadurch gekennzeichnet, dass** es ein Haarfärbemittel ist.

10. Mehrkomponenten-Kit, bestehend aus einer die Verbindung der Formel (I) enthaltenden Farbträgermasse (A1), einer die Kupplersubstanzen und die Persulfatsalze enthaltenden weiteren Farbträgermasse (A2) und einer Wasserstoffperoxid oder dessen Additionsverbindungen enthaltenden wässrigen Zubereitung (A3), sowie ggfs. einem Mittel zur Einstellung des pH-Wertes.

11. Mehrkomponenten-Kit bestehend aus einem die Verbindungen der Formel (I), die Kupplersubstanzen, die Persulfatsalze und gegebenenfalls das Alkalisierungsmittel sowie weitere übliche pulverförmige kosmetische Zusatzstoffe enthaltenden Pulver (Komponente 1), und einer Wasserstoffperoxid und/oder dessen Additionsverbindungen enthaltenden wässrige kosmetische Zubereitung (Komponente 2).

12. Verfahren zum gleichzeitigen Aufhellen und Färben von Haaren bei dem ein Färbemittel nach einem der Ansprüche 1 bis 9 auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird.

## Claims

1. Ready-to-use agent for the simultaneous lightening and dyeing of keratin fibres (A) based on a developer substance-coupler substance combination which has a basic pH, **characterized in that** it comprises (a) at least one heterocyclic hydrazone derivative of the formula (I) or physiologically compatible salt thereof, 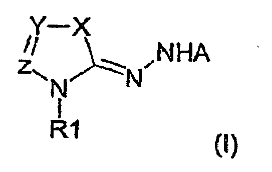 in which
**X** is oxygen, sulphur or N-R2,
**Y** is C-R3 or nitrogen and
**Z** is C-R4 or nitrogen,
with the proviso that the heterocyclic moiety of the compound of the formula (I) contains at most three heteroatoms;
**A** is hydrogen, an acetyl group, a trifluoroacetyl group, a formyl group, a (C₁-C₆)-alkylsulphonyl group or an arylsulphonyl group;
**R1** and **R2** may be identical or different, and, independently of one another, are a saturated or unsaturated (C₁-C₁₂)-alkyl group, a (C₁-C₁₂)-alkyl group substituted by a halogen atom, a hydroxy-(C₁-C₁₂)-alkyl group, an amino- (C₁-C₁₂) -alkyl group, a sulpho-(C₁-C₁₂)-alkyl group, a formyl group, a C(O)-(C₁-C₁₂)-alkyl group, a C(O)-phenyl group, a C(O)NH-alkyl group, a C(O)NH-phenyl group, a substituted or unsubstituted phenyl group or a benzyl group;
**R3** and **R4** may be identical or different and, independently of one another, are hydrogen, a halogen atom, a saturated or unsaturated (C₁-C₁₂)-alkyl group, a (C₁-C₁₂)-alkyl group substituted by a halogen atom, a hydroxy group, a hydroxy-(C₁-C₁₂)-alkyl group, a (C₁-C₁₂)-alkoxy group, a cyano group, a nitro group, an amino group, a (C₁-C₁₂)-alkylamino group, a (C₁-C₁₂)-dialkylamino group, a carboxylic acid, a C(O)O-(C₁-C₁₂)-alkyl group, a substituted or unsubstituted C(O)O-phenyl group, a substituted or unsubstituted phenyl group or a naphthyl group;
and if **Y** and **Z** are C-R3 and C-R4, **R3** and **R4**, together with the remainder of the molecule, form a heterocyclic or carbocyclic, saturated or unsaturated, substituted or unsubstituted ring system;
(b) at least one coupler substance known per se or physiologically compatible salt thereof, and
(c) as oxidizing agent, a combination of at least one persulphate salt and hydrogen peroxide and/or its addition compounds.

2. Agent according to Claim 1, **characterized in that** (i) **X** is sulphur, **Y** is C-R3, **Z** is C-R4 and **A** is a hydrogen atom, or (ii) **X** is N-R2, **Y** is nitrogen and **A** is a hydrogen atom.

3. Agent according to Claim 1 or 2, **characterized in that** the hydrazone derivative of the formula (I) is selected from 3-methyl-2(3H)-thiazolone hydrazone, 3,4-dimethyl-2(3H)-thiazolone hydrazone,
4-tert-butyl-3-methyl-2(3H)-thiazolone hydrazone,
3-methyl-4-phenyl-2(3H)-thiazolone hydrazone,
3-methyl-4-(4-tolyl)-2(3H)-thiazolone hydrazone,
4-(4-methoxy)phenyl-3-methyl-2(3H)-thiazolone hydrazone,
4-(4-ethoxy)phenyl-3-methyl-2(3H)-thiazolone hydrazone,
4-(4-bromophenyl)-3-methyl-2(3H)-thiazolone hydrazone,
4-(3-bromophenyl)-3-methyl-2(3H)-thiazolone hydrazone,
4-(4-chlorophenyl)-3-methyl-2(3H)-thiazolone hydrazone,
4-(3-chlorophenyl)-3-methyl-2(3H)-thiazolone hydrazone,
3-methyl-4-(4-nitrophenyl)-2(3H)-thiazolone hydrazone,
3-methyl-4-(3-nitrophenyl)-2(3H)-thiazolone hydrazone,
4-([1,1'-biphenyl]-4-yl)-3-methyl-2(3H)-thiazolone hydrazone,
3-methyl-4-(2-naphthalenyl)-2(3H)-thiazolone hydrazone,
2-hydrazono-2,3-dihydro-3-methyl-4-thiazolecarboxylic ethyl ester,
3,4,5-trimethyl-2(3H)-thiazolone hydrazone,
3,4-dimethyl-5-phenyl-2(3H)-thiazolone hydrazone,
3,5-dimethyl-4-phenyl-2(3H)-thiazolone hydrazone,
3-methyl-4,5,diphenyl-2(3H)-thiazolone hydrazone,
5-ethyl-3-methyl-4-phenyl-2(3H)-thiazolone hydrazone,
4-(4-bromophenyl)-3-methyl-5-phenyl-2(3H)-thiazolone hydrazone,
3-methyl-5-phenyl-4-(4-tolyl)-2(3H)-thiazolone hydrazone,
5-(4-chlorophenyl)-4-phenyl-3-methyl-2(3H)-thiazol-one hydrazone,
5-(4-chlorophenyl)-4-(4-methoxyphenyl)-3-methyl-2(3H)-thiazolone hydrazone,
2-hydrazono-2,3-dihydro-3,4-dimethyl-4-thiazolecarboxylic ethyl ester,
4-amino-2-hydrazono-2,3-dihydro-3-methyl-5-thiazolecarbonitrile,
3-ethyl-4,5-dimethyl-2(3H)-thiazolone hydrazone,
2-hydrazono-2,3-dihydro-3-ethyl-4-methyl-thiazolecarboxylic ethyl ester,
5-methyl-3-(1-methylethyl)-4-phenyl-2(3H)-thiazolone hydrazone,
4,5-dimethyl-3-(1-methylethyl)-4-phenyl-2(3H)-thiazolone hydrazone,
3-(1-methylethyl)-4,5-diphenyl-2(3H)-thiazolone hydrazone,
4,5-dimethyl-3-propyl-2(3H)-thiazolone hydrazone,
4,5-diphenyl-3-propyl-2(3H)-thiazolone hydrazone,
3-butyl-4,5-dimethyl-2(3H)-thiazolone hydrazone,
3-butyl-4,5-diphenyl-2(3H)-thiazolone hydrazone,
4,5-dimethyl-3-(2-methylpropyl)-2(3H)-thiazolone hydrazone,
3-(2-methylpropyl)-4,5-diphenyl-2(3H)-thiazolone hydrazone,
3-hydroxyethyl-2(3H)-thiazolone hydrazone,
3-hydroxyethyl-4-methyl-2(3H)-thiazolone hydrazone,
3-hydroxyethyl-4,5-dimethyl-2(3H)-thiazolone hydrazone, 3-aminoethyl-2(3H)-thiazolone hydrazone, 3-aminoethyl-4-methyl-2(3H)-thiazolone hydrazone,
3-aminoethyl-4,5-dimethyl-2(3H)-thiazolone hydrazone,
3,4-diphenyl-2(3H)-thiazolone hydrazone,
4-methyl-3-phenyl-2(3H)-thiazolone hydrazone,
4-p-biphenylyl-3-phenyl-2(3H)-thiazolone hydrazone,
4-(4-methoxy)phenyl-3-phenyl-2(3H)-thiazolone hydrazone,
4-tert-butyl-3-phenyl-2(3H)-thiazolone hydrazone,
4,5-dimethyl-3-phenyl-2(3H)-thiazolone hydrazone,
5-methyl-3,4-diphenyl-2(3H)-thiazolone hydrazone,
3,4,5-triphenyl-2(3H)-thiazolone hydrazone,
4,5-dimethyl-3-(phenylmethyl)-2(3H)-thiazolone hydrazone,
3-(2-propenyl)-2(3H)-thiazolone hydrazone,
4-methyl-3-(2-propenyl)-2(3H)-thiazolone hydrazone,
4-tert-butyl-3-(2-propenyl)-2(3H)-thiazolone hydrazone,
4-phenyl-3-(2-propenyl)-2(3H)-thiazolone hydrazone,
4,5-dimethyl-3-(2-propenyl)-2(3H)-thiazolone hydrazone,
4,5-diphenyl-3-(2-propenyl)-2(3H)-thiazolone hydrazone,
4,5-dimethyl-3-(phenylmethyl)-2(3H)-thiazolone hydrazone,
2-hydrazono-2,3-dihydro-3-[(phenylamino)carbonyl]-4-methyl-thiazolecarboxylic ethyl ester,
3-methyl-4,5,6,7-tetrahydro-2(3H)-benzothiazolone hydrazone, 3-methyl-2(3H)-benzothiazolone hydrazone, 3,6-dimethyl-2(3H)-benzothiazolone hydrazone, 6-chloro-3-methyl-2(3H)-benzothiazolone hydrazone, 7-chloro-3-methyl-2(3H)-benzothiazolone hydrazone,
6-hydroxy-3-methyl-2(3H)-benzothiazolone hydrazone,
5-methoxy-3-methyl-2(3H)-benzothiazolone hydrazone,
7-methoxy-3-methyl-2(3H)-benzothiazolone hydrazone,
5,6-dimethoxy-3-methyl-2(3H)-benzothiazolone hydrazone, 5-ethoxy-3-methyl-2(3H)-benzothiazolone hydrazone, 6-ethoxy-3-methyl-2(3H)-benzothiazolone hydrazone, 3-methyl-5-nitro-2(3H)-benzothiazolone hydrazone, 3-methyl-6-nitro-2(3H)-benzothiazolone hydrazone,
5-acetamido-3-methyl-2(3H)-benzothiazolone hydrazone,
6-acetamido-3-methyl-2(3H)-benzothiazolone hydrazone,
5-anilino-3-methyl-2(3H)-benzothiazolone hydrazone,
6-anilino-3-methyl-2(3H)-benzothiazolone hydrazone,
2-hydrazono-2,3-dihydro-3-methyl-6-benzothiazolecarboxylic acid, 2-hydrazono-2,3-dihydro-3-methyl-4-benzothiazolesulphonic acid, 2-hydrazono-2,3-dihydro-3-methyl-5-benzothiazolesulphonic acid, 2-hydrazono-2,3-dihydro-3-methyl-6-benzothiazolesulphonic acid, 2-hydrazono-2,3-dihydro-3-methyl-7-benzothiazolesulphonic acid, 2-hydrazono-2,3-dihydro-N,N,3-trimethyl-6-benzothiazolesulphonamide, [(2-hydrazono-2,3-dihydro-3-methyl-6-benzothiazolyl)oxy]acetic hydrazide, 3-methyl-naphtho[2,3-d]thiazol-2(3H)-one hydrazone, 3-ethyl-2(3H)-benzothiazolone hydrazone, 6-ethoxy-3-ethyl-2(3H)-benzothiazolone hydrazone, 3-propyl-2(3H)-benzothiazolone hydrazone, 3-butyl-2(3H)-benzothiazolone hydrazone, 3-hexyl-2(3H)-benzothiazolone hydrazone, 3-hydroxyethyl-2(3H)-benzothiazolone hydrazone, 3-aminoethyl-2(3H)-benzothiazolone hydrazone, 3-p-methylbenzyl-2(3H)-benzothiazolone hydrazone, 2-hydrazono-2,3-dihydro-3-(2-hydroxyethyl)-6-benzothiazolecarboxylic acid, 2-hydrazono-2,3-dihydro-6-methoxy-3(2H)-benzothiazolepropanesulphonic acid, 6-hexadecyloxy-2-hydrazono-3(2H)-benzothiazolepropanesulphonic acid, 2-oxo-3-benzothiazolineacetic ethyl ester hydrazone, 3-acetyl-2(3H)-benzothiazolone hydrazone, 2-hydrazono-3(2H)-benzothiazole-carboxaldehyde, 3-methyl-2(3H)-oxazolone hydrazone, 3-phenyl-2(3H)-oxazolone hydrazone, 3-methyl-2(3H)benzoxazolone hydrazone, 3-phenyl-2(3H)-benzoxazolone hydrazone, 1,3-dimethyl-4-imidazolin-2-one hydrazone, 1,3-diethyl-4-imidazolin-2-one hydrazone, 1,3-dihydroxyethyl-4-imidazolin-2-one hydrazone, 1,3-diaminoethyl-4-imidazolin-2-one hydrazone, 1,3-dimethyl-4-methoxy-4-imidazolin-2-one hydrazone, 1,3,4-trimethyl-4-imidazolin-2-one hydrazone, 1,3-dimethyl-4-phenyl-4-imidazolin-2-one hydrazone, 4-carboxy-1,3-dimethyl-4-imidazolin-2-one hydrazone, 4-amino-1,3-dimethyl-4-imidazolin-2-one hydrazone, 1,3-dimethyl-4-dimethylamino-4-imidazolin-2-one hydrazone, 1,3-dimethyl-2-benzimidazolinone hydrazone, 1,3-diethyl-2-benzimidazolinone hydrazone, 1,3-dihydroxyethyl-2-benzimidazolinone hydrazone, 1,3-diaminoethyl-2-benzimidazolinone hydrazone, 1,3,5-trimethyl-2-benzimidazolinone hydrazone, 5-methoxy-1,3-dimethyl-2-benzimidazolinone hydrazone, 5-bromo-1,3-dimethyl-2-benzimidazolinone hydrazone, 4,6-dibromo-1,3-dimethyl-2-benzimidazolinone hydrazone, 5-chloro-1,3-dimethyl-2-benzimidazolinone hydrazone, 1,3-dimethyl-5-nitro-2-benzimidazolinone hydrazone, 1,3-dimethyl-6-nitro-2-benzimidazolinone hydrazone, 1,4-dimethyl-Δ2-1,2,4-triazolin-5-one hydrazone, 1,4-dihydroxyethyl-Δ2-1,2,4-triazolin-5-one hydrazone, 1,4-diaminoethyl-Δ2-1,2,4-triazolin-5-one hydrazone, 1,3,4-trimethyl-Δ2-1,2,4-triazolin-5-one hydrazone, 1,4-dimethyl-3-phenyl-Δ2-1,2,4-triazolin-5-one hydrazone, 1,4-dimethyl-3-methoxy-Δ2-1,2,4-triazolin-5-one hydrazone, 1,4-dimethyl-3-dimethylamino-Δ2-1,2,4-triazolin-5-one hydrazone, 4-carboxy-1,4-dimethyl-Δ2-1,2,4-triazolin-5-one hydrazone, 4-amino-1,4-dimethyl-Δ2-1,2,4-triazolin-5-one hydrazone, 4-butyl-1-methyl-3-phenyl-Δ2-1,3,4-triazolin-5-one hydrazone, 4-methyl-Δ2-1,3,4-thiadiazolin-5-one hydrazone, 4-hydroxyethyl-Δ2-1,3,4-thiadiazolin-5-one hydrazone, 4-aminoethyl-Δ2-1,3,4-thiadiazolin-5-one hydrazone, 4-methyl-2-phenyl-Δ2-1,3,4-thiadiazolin-5-one hydrazone, 2-methoxy-4-methyl-Δ2-1,3,4-thiadiazolin-5-one hydrazone, 2-anilino-4-methyl-Δ2-1,3,4-thiadiazolin-5-one hydrazone, 2-amino-4-methyl-Δ2-1,3,4-thiadiazolin-5-one hydrazone, 2-dimethylamino-4-methyl-Δ2-1,3,4-thiadiazolin-5-one hydrazone, 4-methyl-2-(methylthio)-Δ2-1,3,4-thiadiazolin-5-one hydrazone, 4-(5-hydrazono-4,5-dihydro-4-methyl-1,3,4-thiadiazol-2-yl)benzenesulphonyl fluoride, 4-methyl-Δ2-1,2,4-thiadiazolin-5-one hydrazone, 4-hydroxyethyl-Δ2-1,2,4-thiadiazolin-5-one hydrazone, 4-aminoethyl-Δ2-1,2,4-thiadiazolin-5-one hydrazone, 4-methyl-3-phenyl-Δ2-1,2,4-thiadiazolin-5-one hydrazone, 3-methoxy-4-methyl-Δ2-1,2,4-thiadiazolin-5-one hydrazone, 3-amino-4-methyl-Δ2-1,2,4-thiadiazolin-5-one hydrazone, 3-dimethylamino-4-methyl-Δ2-1,2,4-thiadiazolin-5-one hydrazone, 3-carboxy-4-methyl-Δ2-1,2,4-thiadiazolin-5-one hydrazone, 1,4-dimethyl-Δ2, 1,2,4-triazolin-5-one hydrazone, 1,4-dihydroxyethyl-Δ2-1,2,4-triazolin-5-one hydrazone, 1,4-aminoethyl-Δ2-1,2,4-triazolin-5-one hydrazone, 1,3,4-trimethyl-Δ2-1,2,4-triazolin-5-one hydrazone, 1,4-dimethyl-3-phenyl-Δ2-1,2,4-triazolin-5-one hydrazone and 4-methyl-3-phenyl-Δ2-1,2,4-triazolin-5-one hydrazone.

4. Agent according to one of Claims 1 to 3, **characterized in that** the coupler substance is selected from N-(3-dimethylaminophenyl)urea, 2,6-diaminopyridine,
2-amino-4-[(2-hydroxyethyl)amino]anisol,
2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine,
3-amino-6-methoxy-2-(methylamino)pyridine,
2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene,
1,3-diamino-4-(3-hydroxypropoxy)benzene,
1,3-diamino-4-(2-methoxyethoxy)benzene,
2,4-diamino-1,5-di(2-hydroxyethoxy)benzene,
1-(2-aminoethoxy)-2,4-diaminobenzene,
2-amino-1-(2-hydroxyethoxy)-4-methylaminobenzene,
2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole,
3-dimethylaminophenol, 3-diethylaminophenol,
5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol,
3-aminophenol, 2-[(3-hydroxyphenyl)amino]acetamide,- 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol,
5-amino-2-methoxyphenol, 2-(4-amino-2-hydroxyphenoxy)ethanol, 5-[(3-hydroxypropyl)ainino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3;4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

5. Agent according to one of Claims 1 to 4, **characterized in that** the persulphate salt is selected from potassium persulphate, sodium persulphate and ammonium persulphate.

6. Agent according to one of Claims 1 to 5, **characterized in that** it comprises the hydrazone derivatives of the formula (I), and the coupler substances and the persulphate salts in each case in a total amount of from 0.01 to 10 percent by weight.

7. Agent according to one of Claims 1 to 6, **characterized in that** it additionally comprises 0.01 to 10 percent by weight of a physiologically acceptable, direct dye.

8. Agent according to one of Claims 1 to 7, **characterized in that** it has a pH of from 7 to 10.

9. Agent according to one of Claims 1 to 8, **characterized in that** it is a hair colourant.

10. Multicomponent kit consisting of a colour carrier mass (A1) comprising the compound of the formula (I), a further colour carrier mass (A2) comprising the coupler substances and the persulphate salts, and an aqueous preparation (A3) comprising hydrogen peroxide or its addition compounds, and also optionally an agent for adjusting the pH.

11. Multicomponent kit consisting of a powder (component 1) comprising the compounds of the formula (I), the coupler substances, the persulphate salts and optionally the alkalizing agent, and further customary pulverulent cosmetic additives, and an aqueous cosmetic preparation (component 2) comprising hydrogen peroxide and/or its addition compounds.

12. Method for the simultaneous lightening and dyeing of hair in which a colourant according to one of Claims 1 to 9 is applied to the hair and, after a contact time of from 5 to 60 minutes at a temperature of from 20 to 50°C, the hair is rinsed with water, optionally washed with a shampoo and then dried.

## Revendications

1. Composition prête à l'emploi pour l'éclaircissement et la teinture simultanés de fibres de kératine (A) à base d'une association développeur-coupleur, composition qui présente un pH basique, **caractérisée en ce qu'**elle contient (a) au moins un dérivé d'hydrazone hétérocyclique de formule (I) ou un sel physiologiquement acceptable d'un tel dérivé, 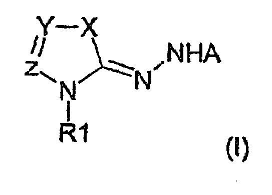 formule dans laquelle
**X** est un atome d'oxygène ou de soufre ou N-R2,
**Y** est C-R3 ou un atome d'azote et
**Z** est C-R4 ou un atome d'azote,
avec la condition que la partie hétérocyclique du composé de formule (I) contient au maximum trois hétéroatomes ;
**A** représente un atome d'hydrogène, un groupe acétyle, un groupe trifluoroacétyle, un groupe formyle, un groupe alkyl(C₁-C₆)sulfonyle ou un groupe arylsulfonyle ;
**R1** et **R2** peuvent être identiques ou différents et représentent, indépendamment l'un de l'autre, un groupe alkyle en C₁-C₁₂ saturé ou insaturé, un groupe alkyle en C₁-C₁₂ substitué par un atome d'halogène, un groupe hydroxyalkyle(C₁-C₁₂), un groupe aminoalkyle(C₁-C₁₂), un groupe sulfoalkyle(C₁-C₁₂), un groupe formyle, un groupe C(O)-alkyle(C₁-C₁₂), un groupe C(O)-phényle, un groupe C(O)NH-alkyle, un groupe C(O)NH-phényle, un groupe phényle substitué ou non substitué ou un groupe benzyle ;
**R3** et **R4** peuvent être identiques ou différents et représentent, indépendamment l'un de l'autre, un atome d'hydrogène, un atome d'halogène, un groupe alkyle en C₁-C₁₂ saturé ou insaturé, un groupe alkyle en C₁-C₁₂ substitué par un atome d'halogène, un groupe hydroxyalkyle(C₁-C₁₂), un groupe alcoxy en C₁-C₁₂, un groupe cyano, un groupe nitro, un groupe amino, un groupe alkyl(C₁-C₁₂)amino, un groupe dialkyl(C₁-C₁₂)amino, un acide carboxylique, un groupe C(O)O-alkyle(C₁-C₁₂)_{,} un groupe C(O)O-phényle substitué ou non substitué, un groupe phényle substitué ou non substitué ou un groupe naphtyle ;
et lorsque **Y** et **Z** représentent C-R3 et C-R4, **R3** et **R4** forment ensemble avec la molécule restante un système cyclique hétérocyclique ou carbocyclique, saturé ou insaturé, substitué ou non substitué ;
(b) au moins un coupleur connu en soi ou un sel physiologiquement acceptable d'un tel coupleur, et
(c) en tant qu'oxydant une association d'au moins un persulfate et de peroxyde d'hydrogène et/ou de ses composés d'addition.

2. Composition selon la revendication 1, **caractérisée en ce que** (i) **X** représente un atome de soufre, **Y** représente C-R3, **Z** représente C-R4 et **A** représente un atome d'hydrogène, ou (ii) **X** représente N-R2, **Y** est un atome d'azote et **A** représente un atome d'hydrogène.

3. Composition selon la revendication 1 ou 2, **caractérisée en ce que** le dérivé d'hydrazone de formule (I) est choisi parmi la 3-méthyl-2(3H)-thiazolone-hydrazone, la 3,4-diméthyl-2(3H)-thiazolone-hydrazone, la 4-tert-butyl-3-méthyl-2(3H)-thiazolone-hydrazone, la 3-méthyl-4-phényl-2(3H)-thiazolone-hydrazone, la 3-méthyl-4-(4-tolyl)-2(3H)-thiazolone-hydrazone, la 4-(4-méthoxy)phényl-3-méthyl-2(3H)-thiazolone-hydrazone, la 4-(4-éthoxy)phényl-3-méthyl-2(3H)-thiazolone-hydrazone, la 4-(4-bromophényl)-3-méthyl-2(3H)-thiazolone-hydrazone, la 4-(3-bromophényl)-3-méthyl-2(3H)-thiazolone-hydrazone,
la 4-(4-chlorophényl)-3-méthyl-2(3H)-thiazolone-hydrazone, la 4-(3-chlorophényl)-3-méthyl-2(3H)-thiazolone-hydrazone, la 3-méthyl-4-(4-nitro-phényl)-2(3H)-thiazolone-hydrazone, la 3-méthyl-4-(3-nitrophényl)-2(3H)-thiazolone-hydrazone, la 4-([1,1'-biphényl]-4-yl)-3-méthyl-2(3H)-thiazolone-hydrazone, la 3-méthyl-4-(2-naphtalényl)-2(3H)-thiazolone-hydrazone, le 2-hydrazono-2,3-dihydro-3-méthyl-4-thiazolecarboxylate d'éthyle, la 3,4,5-triméthyl-2(3H)-thiazolone-hydrazone, la 3,4-diméthyl-5-phényl-2(3H)-thiazolone-hydrazone, la 3,5-diméthyl-4-phényl-2(3H)-thiazolone-hydrazone,
la 3-méthyl-4,5-diphényl-2(3H)-thiazolone-hydrazone, la 5-éthyl-3-méthyl-4-phényl-2(3H)-thiazolone-hydrazone, la 4-(4-bromophényl)-3-méthyl-5-phényl-2(3H)-thiazolone-hydrazone, la 3-méthyl-5-phényl-4-(4-tolyl)-2(3H)-thiazolone-hydrazone, la 5-(4-chlorophényl)-4-phényl-3-méthyl-2(3H)-thiazolone-hydrazone, la 5-(4-chlorophényl)-4-(4-méthoxyphényl)-3-méthyl-2(3H)-thiazolone-hydrazone, le 2-hydrazono-2,3-dihydro-3,4-diméthyl-4-thiazolecarboxylate d'éthyle, le 4-amino-2-hydrazono-2,3-dihydro-3-méthyl-5-thiazolecarbonitrile, la 3-éthyl-4,5-diméthyl-2(3H)-thiazolone-hydrazone, le 2-hydrazono-2,3-dihydro-3-éthyl-4-méthyl-thiazolecarboxylate d'éthyle, la 5-méthyl-3-(1-méthyléthyl)-4-phényl-2(3H)-thiazolone-hydrazone, la 4,5-diméthyl-3-(1-méthyléthyl)-4-phényl-2(3H)-thiazolone-hydrazone,
la 3-(1-méthyléthyl)-4,5-diphényl-2(3H)-thiazolone-hydrazone, la 4,5-diméthyl-3-propyl-2(3H)-thiazolone-hydrazone, la 4,5-diphényl-3-propyl-2(3H)-thiazolone-hydrazone, la 3-butyl-4,5-diméthyl-2(3H)-thiazolone-hydrazone, la 3-butyl-4,5-diphényl-2(3H)-thiazoloné-hydrazone, la 4,5-diméthyl-3-(2-méthylpropyl)-2(3H)-thiazolone-hydrazone, la 3-(2-méthylpropyl)-4,5-diphényl-2(3H)-thiazolone-hydrazone, la 3-hydroxyéthyl-2(3H)-thiazolone-hydrazone, la 3-hydroxyéthyl-4-méthyl-2(3H)-thiazolone-hydrazone, la 3-hydroxyéthyl-4,5-diméthyl-2(3H)-thiazolone-hydrazone, la 3-aminoéthyl-2(3H)-thiazolone-hydrazone, la 3-aminoéthyl-4-méthyl-2.(3H)-thiazolone-hydrazone, la 3-aminoéthyl-4,5-diméthyl-2(3H)-thiazolone-hydrazone, la 3,4-diphényl-2(3H)-thiazolone-hydrazone, la 4-méthyl-3-phényl-2(3H)-thiazolone-hydrazone, la 4-p-biphénylyl-3-phényl)-2(3H)-thiazolone-hydrazone,
la 4-(4-méthoxy)phényl-3-phényl)-2(3H)-thiazolone-hydrazone, la 4-tert-butyl-3-phényl)-2(3H)-thiazolone-hydrazone, la 4,5-diméthyl-3-phényl-2(3H)-thiazolone-hydrazone, la 5-méthyl-3,4-diphényl-2(3H)-thiazolone-hydrazone, la 3,4,5-triphényl-2(3H)-thiazolone-hydrazone, la 4,5-diméthyl-3-(phénylméthyl)-2(3H)-thiazolone-hydrazone, la 3-(2-propényl)-2(3H)-thiazolone-hydrazone, la 4-méthyl-3-(2-propényl)-2(3H)-thiazolone-hydrazone, la 4-tert-butyl-3-(2-propényl)-2(3H)-thiazolone-hydrazone, la 4-phényl-3-(2-propényl)-2(3H)-thiazolone-hydrazone, la 4,5-diméthyl-3-(2-propényl)-2(3H)-thiazolone-hydrazone, la 4,5-diphényl-3-(2-propényl)-2(3H)-thiazolone-hydrazone, la 4,5-diméthyl-3-(phénylméthyl)-2(3H)-thiazolone-hydrazone, le 2-hydrazono-2,3-dihydro-3-[(phénylamino)carbonyl]-4-méthyl-thiazolecarboxylate d'éthyle, la 3-méthyl-4,5,6,7-tétrahydro-2(3H)-benzothiazolone-hydrazone, la 3-méthyl-2(3H)-benzothiazolone-hydrazone, la 3,6-diméthyl-2(3H)-benzothiazolone-hydrazone, la 6-chloro-3-méthyl-2(3H)-benzothiazolone-hydrazone, la 7-chloro-3-méthyl-2 (3H)-benzothiazolone-hydrazone, la 6-hydroxy-3-méthyl-2(3H)-benzothiazolone-hydrazone, la 5-méthoxy-3-méthyl-2(3H)-benzothiazolone-hydrazone,
la 7-méthoxy-3-méthyl-2(3H)-benzothiazolone-hydrazone, la 5,6-diméthoxy-3-méthyl-2(3H)-benzothiazolone-hydrazone, la 5-éthoxy-3-méthyl-2(3H)-benzothiazolorie-hydrazone, la 6-éthoxy-3-méthyl-2(3H)-benzothiazolone-hydrazone, la 3-méthyl-5-nitro-2(3H)-benzothiazolone-hydrazone, la 3-méthyl-6-nitro-2 (3H)-benzothiazolone-hydrazone,
la 5-acétamido-3-méthyl-2(3H)-benzothiazolone-hydrazone, la 6-acétamido-3-méthyl-2(3H)-benzothiazolone-hydrazone, la 5-anilino-3-méthyl-2(3H)-benzothiazolone-hydrazone, la 6-anilino-3-méthyl-2(3H)-benzothiazolone-hydrazone, l'acide 2-hydrazono-2,3-dihydro-3-méthyl-6-benzothiazole-carboxylique, l'acide 2-hydrazono-2,3-dihydro-3-méthyl-4-benzothiazole-sulfonique, l'acide 2-hydrazono-2,3-dihydro-3-méthyl-5-benzothiazole-sulfonique, l'acide 2-hydrazono-2,3-dihydro-3-méthyl-6-benzothiazole-sulfonique, l'acide 2-hydrazono-2,3-dihydro-3-méthyl-7-benzothiazole-sulfonique, le 2-hydrazono-2,3-dihydro-N,N,3-triméthyl-6-benzothiazole-sulfonamide, le [(2-hydrazono-2,3-dihydro-3-méthyl-6-benzothiazolyl)-oxy]acéto-hydrazide, la 3-méthyl-naphto[2,3-d]-thiazol-2(3H)-one-hydrazone, la 3-éthyl-2(3H)-benzothiazolone-hydrazone, la 6-éthoxy-3-éthyl-2(3H)-benzothiazolone-hydrazone, la 3-propyl-2(3H)-benzothiazolone-hydrazone, la 3-butyl-2(3H)-benzothiazolone-hydrazone, la 3-hexyl-2(3H)-benzothiazolone-hydrazone, la 3-hydroxyéthyl-2(3H)-benzothiazolone-hydrazone, la 3-aminoéthyl-2(3H)-benzothiazolone-hydrazone, la 3-p-méthyl-benzyl-2(3H)-benzothiazolone-hydrazone, l'acide 2-hydrazono-2,3-dihydro-3-(2-hydroxyéthyl)-6-benzothiazole-carboxylique, l'acide 2-hydrazono-2,3-dihydro-6-méthoxy-3(2H)benzothiazole-propane-sulfonique, l'acide 6-hexadécyloxy-2-hydrazono-3(2H)-benzothiazole-propanesulfonique, la 2-oxo-3-benzothiazoline-acétate d'éthyle-hydrazone, la 3-acetyl-2(3H)-benzothiazolone-hydrazone, le 2-hydrazono-3(2H)-benzothiazole-carboxaldéhyde, la 3-méthyl-2(3H)-oxazolone-hydrazone, la 3-phényl-2(3H)-oxazolone-hydrazone, la 3-méthyl-2(3H)-benzoxazolone-hydrazone, la 3-phényl-2(3H)-benzoxazolone-hydrazone, la 1,3-diméthyl-4-imidazolin-2-one-hydrazone, la 1,3-diéthyl-4-imidazolin-2-one-hydrazone, la 1,3-dihydroxyéthyl-4-imidazolin-2-one-hydrazone, la 1,3-diaminoéthyl-4-imidazolin-2-one-hydrazone, la 1,3-diméthyl-4-méthoxy-4-imidazolin-2-one-hydrazone, la 1,3,4-triméthyl-4-imidazolin-2-one-hydrazone, la 1,3-diméthyl-4-phényl-4-imidazolin-2-one-hydrazone, la 4-carboxy-1,3-diméthyl-4-imidazolin-2-one-hydrazone, la 4-amino-1,3-diméthyl-4-imidazolin-2-one-hydrazone, la 1,3-diméthyl-4-diméthylamino-4-imidazolin-2-one-hydrazone, la 1,3-diméthyl-2-benzimidazolinone-hydrazone, la 1,3-diéthyl-2-benzimidazolinone-hydrazone, la 1,3-dihydroxy-éthyl-2-benzimidazolinone-hydrazone, la 1,3-diaminoéthyl-2-benzimidazolinone-hydrazone, la 1,3,5-triméthyl-2-benzimidazolinone-hydrazone, la 5-méthoxy-1,3-diméthyl-2-benzimidazolinone-hydrazone, la 5-bromo-1,3-diméthyl-2-benzimidazolinone-hydrazone, la 4,6-dibromo-1,3-diméthyl-2-benzimidazolinone-hydrazone, la 5-chloro-1,3-diméthyl-2-benzimidazolinone-hydrazone, la 1,3-diméthyl-5-nitro-2-benzimidazolinone-hydrazone, la 1,3-diméthyl-6-nitro-2-benzimidazolinone-hydrazone, la 1,4-diméthyl-A2-1,2,4-triazolin-5-one-hydrazone, la 1,4-dihydroxyéthyl-Δ2-1,2,4-triazolin-5-one-hydrazone,
la 1,4-diaminoéthyl-Δ2-1,2,4-triazolin-5-one-hydrazone, la 1,3,4-triméthyl-à2-1,2,4-triazolin-5-one-hydrazone, la 1,4-diméthyl-3-phényl-Δ2-1,2,4-triazolin-5-one-hydrazone, la 1,4-diméthyl-3-méthoxy-Δ2-1,2,4-triazolin-5-one-hydrazone, la 1,4-diméthyl-3-diméthylamino-Δ2-1,2,4-triazolin-5-one-hydrazone, la 4-carboxy-1,4-diméthyl-Δ2-1,2,4-triazolin-5-one-hydrazone, la 4-amino-1,4-diméthyl-Δ2-1,2,4-triazolin-5-one-hydrazone, la 4-butyl-1-méthyl-3-phényl-Δ2-1,3,4-triazolin-5-one-hydrazone, la 4-méthyl-Δ2-1,3,4-thiadiazolin-5-one-hydrazone, la 4-hydroxyéthyl-Δ2-1,3,4-thiadiazolin-5-one-hydrazone, la 4-aminoéthyl-Δ2-1,3,4-thiadiazolin-5-one-hydrazone, la 4-méthyl-2-phényl-Δ2-1,3,4-thiadiazolin-5-one-hydrazone, la 2-méthoxy-4-méthyl-Δ2-1,3,4-thiadiazolin-5-one-hydrazone, la 2-anilino-4-méthyl-Δ2-1,3,4-thiadiazolin-5-one-hydrazone, la 2-amino-4-méthyl-Δ2-1,3,4-thiadiazolin-5-one-hydrazone, la 2-diméthylamino-4-méthyl-Δ2-1,3,4-thiadiazolin-5-one-hydrazone, la 4-méthyl-2-(méthylthio)-Δ2-1,3,4-thiadiazolin-5-one-hydrazone, le fluorure de 4-(5-hydrazono-4,5-dihydro-4-méthyl-1,3,4-thiadiazol-2-yl)-benzènesulfonyle, la 4-méthyl-Δ2-1,2,4-thiadiazolin-5-one-hydrazone, la 4-hydroxyéthyl-à2-1,2,4-thiadiazolin-5-one-hydrazone, la 4-aminoéthyl-Δ2-1,2,4-thiadiazolin-5-one-hydrazone, la 4-méthyl-3-phényl-Δ2-1,2,4-thiadiazolin-5-one-hydrazone, la 3-méthoxy-4-méthyl-Δ2-1,2,4-thiadiazolin-5-one-hydrazone, la 3-amino-4-méthyl-Δ2-1,2,4-thiadiazolin-5-one-hydrazone, la 3-diméthylamino-4-méthyl-Δ2-1,2,4-thiadiazolin-5-one-hydrazone, la 3-carboxy-4-méthyl-Δ2-1,2,4-thiadiazolin-5-one-hydrazone, la 1,4-diméthyl-Δ2-1,2,4-triazolin-5-one-hydrazone,
la 1,4-dihydroxyéthyl-Δ2-1,2,4-triazolin-5-one-hydrazone, la 1,4-aminoéthyl-Δ2-1,2,4-triazolin-5-one-hydrazone, la 1,3,4-triméthyl-Δ2-1,2,4-triazolin-5-one-hydrazone, la 1,4-diméthyl-3-phényl-Δ2-1,2,4-triazolin-5-one-hydrazone, et la 4-méthyl-3-phényl-Δ2-1,2,4-triazolin-5-one-hydrazone.

4. Composition selon l'une quelconque des revendications 1 à 3, **caractérisée en ce que caractérisée en ce que** le coupleur est choisi parmi la N-(3-diméthylaminophényl)urée, la 2,6-diaminopyridine, le 2-amino-4-[(2-hydroxyéthyl)-amino]anisole, le 2,4-diamino-1-fluoro-5-méthyl-benzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxy-benzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 1,3-diamino-4-(2,3-dihydroxypropoxy)benzène, le 1,3-diamino-4-(3-hydroxypropoxy)benzène, le 1,3-diamino-4-(2-méthoxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)-amino]aniline, le 4-amino-2-di[(2-hydroxyéthyl)-amino]-1-éthoxybenzène, le 5-méthyl-2-(1-méthyl-éthyl)phénol, la 3-[(2-hydroxyéthyl)amino]aniline, la 3-[(2-aminoéthyl)amino]aniline, le 1,3-di(2,4-diaminophénoxy)propane, le di(2,4-diaminophénoxy)-méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]-acétamide, le 5-[(2-hydroxyéthyl)amino]-4-méthoxy-2-méthylphénol, le 5-[(2-hydroxyéthyl)amino]-2-méthylphénol, le 3-[(2-hydroxyéthyl)amino]phénol, 3-[(2-méthoxyéthyl)amino]phénol, le 5-amino-2-éthylphénol, le 5-amino-2-méthoxyphénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthyl-phénol, la 2-amino-3-hydroxypyridine, la 2,6-dihydroxy-3,4-diméthylpyridine, le 5-amino-4-chloro-2-méthylphénol, le 1-naphtol, le 2-méthyl-1-naphtol, le 1,5-dihydroxynaphthalène, le 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro-2,4-dihydroxybenzène, le 2-chloro-1,3-dihydroxybenzène, 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)-amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

5. Composition selon l'une quelconque des revendications 1 à 4, **caractérisée en ce que** le persulfate est choisi parmi le persulfate de potassium, le persulfate de sodium et le persulfate d'ammonium.

6. Composition selon l'une quelconque des revendications 1 à 5, **caractérisée en ce qu'**elle contient les dérivés d'hydrazone de formule (I) ainsi que les coupleurs et les persulfates chacun en une quantité totale de 0,01 à 10% en poids.

7. Composition selon l'une quelconque des revendications 1 à 6, **caractérisée en ce qu'**elle contient en outre de 0,01 à 10% en poids d'un colorant direct physioloiquement sans inconvénient.

8. Composition selon l'une quelconque des revendications 1 à 7, **caractérisée en ce qu'**elle présente un pH de 7 à 10.

9. Composition selon l'une quelconque des revendications 1 à 8, **caractérisée en ce qu'**elle est une composition de teinture pour cheveux.

10. Nécessaire à plusieurs composants, constitué d'une matière colorante (A1) contenant le composé de formule (I), d'une autre matière colorante (A2) contenant les coupleurs et les persulfates et d'une préparation aqueuse (A3) contenant du peroxyde d'hydrogène ou ses composés d'addition, ainsi qu'éventuellement d'un agent pour l'ajustement du pH.

11. Nécessaire à plusieurs composants, constitué d'une poudre (composant 1) contenant les composés de formule (I), les coupleurs, les persulfates et éventuellement l'agent d'alcalinisation ainsi que d'autres additifs cosmétiques pulvérulents usuels, et d'une préparation cosmétique aqueuse (composant 2) contenant du peroxyde d'hydrogène et/ou ses composés d'addition.

12. Procédé pour l'éclaircissement et la teinture simultanés des cheveux, dans lequel on applique sur les cheveux une composition de teinture selon l'une quelconque des revendications 1 à 9 et, après un temps d'action de 5 à 60 minutes à une température de 20 à 50 °C, on rince les cheveux à l'eau, éventuellement on les lave avec un shampooing et ensuite on les sèche.
